# EUROPEAN PATENT SPECIFICATION

(11) **EP 4 132 515 B1**
(45) Date of publication and mention of the grant of the patent: **05.08.2026**
(21) Application number: 21716907.7
(22) Date of filing: 12.03.2021
(51) Int. Cl.: A61K 31/417, A61K 39/00, A61K 45/06, A61P 25/28, A61P 35/00, A61K 40/11, A61K 40/31, A61K 40/42

(54) **A COMBINATION THERAPY WITH NIROGACESTAT AND A BCMA-DIRECTED THERAPY AND USES THEREOF**
KOMBINATIONSTHERAPIE MIT NIROGECESSTAT UND EINER BCMA-GERICHTETEN THERAPIE UND VERWENDUNGEN DAVON
POLYTHÉRAPIE AVEC DU NIROGACESTAT ET UNE THÉRAPIE DIRIGÉE CONTRE BCMA ET LEURS UTILISATIONS

(30) Priority: 13.03.2020 US 202062989372 P
(43) Date of publication of application: 15.02.2023
(73) Proprietor: Springworks Therapeutics, Inc., Stamford, CT 06902 (US)
(72) Inventor: SHEARER, Todd Webster, Stamford, CT 06902 (US); EDRIS, Badreddin, Stamford, CT 06902 (US)
(74) Representative: Pohlman, Sandra M.
(86) International application number: PCT/US2021/022177
(87) International publication number: WO 2021/183934

(56) References cited:
- WO-A1-2018/151836
- WO-A1-2018/201051
- WO-A1-2018/201056
- WO-A1-2019/090364
- WO-A1-2020/208572
- WO-A1-2021/029854
- US-A1- 2016 354 382
- ANONYMOUS: "Platform Study of Belantamab Mafodotin as Monotherapy and in Combination With Anti-cancer Treatments in Participants With Relapsed/Refractory Multiple Myeloma (RRMM) (DREAMM 5) - Full Text View - ClinicalTrials.gov", CLINICAL TRIALS.GOV, 15 October 2019 (2019-10-15), XP055685653, Retrieved from the Internet <URL:https://clinicaltrials.gov/ct2/show/NCT04126200?term=GSK3174998+GSK2857916&draw=2&rank=1> [retrieved on 20200415]
- EASTMAN S ET AL: "Synergistic Activity of Belantamab Mafodotin (anti-BCMA immuno-conjugate) with PF-03084014 (gamma-secretase inhibitor) in Bcma-Expressing Cancer Cell Lines | Blood | American Society of Hematology", 1 November 2019 (2019-11-01), XP055810995, Retrieved from the Internet <URL:https://ashpublications.org/blood/article/134/Supplement_1/4401/424544/Synergistic-Activity-of-Belantamab-Mafodotin-anti> [retrieved on 20210607]

## Description

### FIELD OF THE INVENTION

The present disclosure provides methods of treating cancer (e.g., multiple myeloma) or light chain amyloidosis in a subject in need thereof comprising administering a combination therapy comprising an effective amount of Form A of nirogacestat dihydrobromide and a B-cell maturation antigen (BCMA)-directed therapy to the subject.

### BACKGROUND

B-cell maturation antigen (BCMA) is a substrate of gamma secretase (Laurent et al., Nat Commun. 2015 Jun 11, 6:7333). Gamma secretase is a multi-subunit protease complex that cleaves single-pass transmembrane proteins at residues within the transmembrane domain. BCMA expression has been linked to a number of cancers, including hematological cancers, such as multiple myeloma.

WO 2018/201056 A1 relates combination therapies of a BCMA CAR-expressing cell and a gamma secretase inhibitor and to the treatment of diseases associated with expression of BCMA.

There is a need for improved strategies for targeting diseases, such as cancer or light chain amyloidosis, and methods for improving existing therapeutic agents that target BCMA.

### BRIEF SUMMARY OF THE INVENTION

The present invention is defined by the claims. References to methods of treatment in this description are to be interpreted as references to compounds, combination of compounds, combination therapies, or compositions of the present invention for use in those methods. The present disclosure relates to a method of treating cancer in a subject in need thereof comprising administering a combination therapy comprising an effective amount of Form A of nirogacestat dihydrobromide and a BCMA-directed therapy to the subject. In one aspect, the present disclosure relates to the use of a combination therapy comprising an effective amount of Form A of nirogacestat dihydrobromide and a BCMA-directed therapy in treating cancer in a subject in need thereof.

In one aspect, the present invention pertains to a combination therapy comprising nirogacestat dihydrobromide and a B-cell maturation antigen (BCMA)-directed therapy for use in treating cancer in a subject in need thereof, wherein the nirogacestat dihydrobromide is in Form A and exhibits an XRPD pattern having peaks at 8.8 ± 0.2, 9.8 ± 0.2, and 23.3 ± 0.2 degrees two-theta.

In one aspect, the cancer is characterized by inadequate expression of BCMA.

In another aspect, the cancer is characterized by detectable soluble BCMA levels in a serum sample from the subject.

In another aspect, the cancer is a hematologic cancer. In one aspect, the hematologic cancer is multiple myeloma. In one aspect, the cancer is selected from a group consisting of Waldenstrom macroglobulinemia, chronic lymphocytic leukemia (CLL), diffuse large B cell lymphoma (DLBCL), follicular lymphoma (FL), Burkitt lymphoma (BL), mantle cell lymphoma (MCL), and myelogenous leukemia (ML).

In another aspect, the present disclosure relates to a method of treating light chain amyloidosis comprising administering a combination therapy comprising an effective amount of Form A of nirogacestat dihydrobromide and a BCMA-directed therapy to a subject in need thereof. In another aspect, the present disclosure relates to the use of a combination therapy comprising an effective amount of Form A of nirogacestat dihydrobromide and a BCMA-directed therapy in treating light chain amyloidosis in a subject in need thereof.

In one aspect, the present invention pertains to a combination therapy comprising nirogacestat dihydrobromide and a B-cell maturation antigen (BCMA)-directed therapy for use in treating light chain amyloidosis in a subject in need thereof, the wherein nirogacestat dihydrobromide is in Form A and exhibits an XRPD pattern having peaks at 8.8 ± 0.2, 9.8 ± 0.2, and 23.3 ± 0.2 degrees two-theta.

In one aspect, the Form A of nirogacestat dihydrobromide reduces the shedding of BCMA from the surface of a BCMA positive cell in the subject.

In another aspect, the Form A of nirogacestat dihydrobromide reduces the levels of soluble BCMA in the subject.

In another aspect, the Form A of nirogacestat dihydrobromide increases the percentage of BCMA-positive cancer cells in the subject.

In another aspect, the Form A of nirogacestat dihydrobromide increases the BCMA density of membrane bound BCMA on the surface of BCMA-positive cancer cells in the subject.

In another aspect, the Form A of nirogacestat dihydrobromide enhances the activity of BCMA-directed therapy in the subject.

In another aspect, the Form A of nirogacestat dihydrobromide enables administration of a lower dose of BCMA-directed therapy to the subject as compared with the amount of the BCMA-directed therapy administered alone while maintaining equal levels of efficacy (*e.g.,* one or more of the treatment endpoints discussed below (*e.g.,* CR, nCR, sCR, MRD)). In some aspects, the Form A of nirogacestat dihydrobromide enables administration of a lower dose or the same dose of BCMA-directed therapy to the subject as compared with the amount of the BCMA-directed therapy administered alone while achieving increased levels of efficacy (*e.g.,* one or more of the treatment endpoints discussed below (*e.g.,* CR, nCR, sCR, MRD)).

In one aspect, the subject is administered the Form A of nirogacestat dihydrobromide at a dose from about 20 mg to about 220 mg. In another aspect, the subject is administered the Form A of nirogacestat dihydrobromide at a dose from about 20 mg to about 220 mg once or twice daily. In another aspect, the subject is administered the Form A of nirogacestat dihydrobromide at a dose from about 20 mg to about 220 mg once or twice daily for at least one week.

In one aspect, the Form A of nirogacestat dihydrobromide is administered to the subject before, concomitantly, or subsequently to the administering of the BCMA-directed therapy to the subject.

In one aspect, the subject is administered the combination therapy as a first line of therapy.

In one aspect, the subject with cancer or light chain amyloidosis being treated with Form A of nirogacestat dihydrobromide and a BCMA-directed therapy has been previously treated for the cancer or light chain amyloidosis. In some aspects, the subject with cancer or light chain amyloidosis being treated with Form A of nirogacestat dihydrobromide and a BCMA-directed therapy has been previously treated for the cancer or light chain amyloidosis with one or more of a proteasome inhibitor, an immunomodulatory therapy, an immunotherapy (e.g., a monoclonal antibody, such as a monoclonal antibody directed to CD38), a stem cell transplant, a chemotherapy, a targeted therapy *(e.g.,* an XPO1 inhibitor), or a BCMA-directed therapy not in combination with nirogacestat.

In one aspect, the Form A of nirogacestat dihydrobromide is administered orally and the BCMA-directed therapy is administered intravenously or subcutaneously to the subject. In one aspect, the BCMA-directed therapy includes one or more of an allogeneic chimeric antigen receptor T cell therapy, an autologous chimeric antigen receptor T cell therapy, an immunotherapy (e.g., a monoclonal antibody therapy), an antibody drug conjugate therapy, or a bispecific antibody therapy with dual specificity for BCMA and an immune-related target (*e.g.,* CD3). In another aspect, the BCMA-directed therapy includes at least an allogeneic chimeric antigen receptor T cell therapy. In another aspect, the BCMA-directed therapy includes at least an autologous chimeric antigen receptor T cell therapy. In another aspect, the BCMA-directed therapy includes at least an immunotherapy (*e.g.,* a monoclonal antibody therapy). In another aspect, the BCMA-directed therapy includes at least an antibody drug conjugate therapy. In another aspect, the BCMA-directed therapy includes at least a bispecific antibody therapy with dual specificity for BCMA and an immune-related target (*e.g.,* CD3).

In one aspect, the Form A of nirogacestat dihydrobromide is administered in a tablet form.

In one aspect, the subject is human.

### BRIEF DESCRIPTION OF THE FIGURES

Figure 1 is a powder X-ray diffraction pattern ("XRPD") corresponding to crystalline Form A.
Figure 2 is a thermogravimetric analysis thermogram ("TGA") corresponding to crystalline Form A.
Figure 3 is a differential scanning calorimetry thermogram ("DSC") corresponding to crystalline Form A.

### DETAILED DESCRIPTION OF THE INVENTION

### I. Overview

B-cell maturation antigen (BCMA) is expressed on the surface of plasma cells and regulates their survival. In multiple myeloma, BCMA is widely expressed on malignant cells but is largely not expressed on normal tissues. BCMA can be released from the cell surface as soluble BCMA (sBCMA) which can be detected in the serum of patients with several different types of B cell malignancies. Levels of BCMA in the serum can be correlated with disease activity and overall survival of these patients.

Gamma secretase is involved in the cleavage of membrane bound BCMA and shedding of the BCMA's extracellular domain into the serum as soluble BCMA. BCMA shedding can create challenges for therapeutic agents that target BCMA. Some of the challenges include the following. First, BCMA shedding can decrease surface BCMA expression on cancer cells which then reduces target binding sites for BCMA-targeting therapeutic agents. Second, BCMA shedding can generate a soluble BCMA sink that binds to BCMA-targeting therapeutic agents and diverts these agents from binding to membrane bound BCMA expressed on cancer cells. Third, soluble BCMA molecules can also sequester circulating BCMA ligands, *e.g.,* B-cell activating factor (BAFF) and a proliferation-inducing ligand (APRIL), and prevent them from stimulating BCMA expressed on the surface of B cells and plasma cells, thereby leading to deficient humoral immune responses in patients.

The use of gamma secretase inhibitors to prevent BCMA shedding can increase the effectiveness of BCMA-directed therapies that target pathological B cells expressing BCMA. The present disclosure provides a method of treating cancer or light chain amyloidosis in a subject in need thereof comprising administering a combination therapy comprising an effective amount of Form A of nirogacestat dihydrobromide and a BCMA-directed therapy to the subject. In one aspect, the cancer is characterized by inadequate expression of BCMA.

In another aspect, the cancer is characterized by detectable sBCMA levels.

In another aspect, the cancer is a hematologic cancer. In one aspect, the hematologic cancer is multiple myeloma. In one aspect, the cancer is selected from a group consisting of Waldenstrom macroglobulinemia, chronic lymphocytic leukemia (CLL), diffuse large B cell lymphoma (DLBCL), follicular lymphoma (FL), Burkitt lymphoma (BL), mantle cell lymphoma (MCL), and myelogenous leukemia (ML).

In one aspect, the present disclosure provides a method of treating light chain amyloidosis a subject in need thereof comprising administering a combination therapy comprising an effective amount of Form A of nirogacestat dihydrobromide and a BCMA-directed therapy to the subject.

### II. Definitions

To facilitate an understanding of the disclosure set forth herein, a number of terms and phrases are defined below.

Generally, the nomenclature used herein and the laboratory procedures in organic chemistry, medicinal chemistry, and pharmacology described herein are those well-known and commonly employed in the art. Unless defined otherwise, all technical and scientific terms used herein generally have the same meaning as commonly understood by one of ordinary skill in the art to which this disclosure belongs.

In this specification and the appended claims, the singular forms "a," "an" and "the" include plural referents unless the context clearly dictates otherwise. The terms "a" (or "an"), as well as the terms "one or more," and "at least one" can be used interchangeably herein. In certain aspects, the term "a" or "an" means "single." In other aspects, the term "a" or "an" includes "two or more" or "multiple."

Furthermore, "and/or" where used herein is to be taken as specific disclosure of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

The term "subject" refers to an animal, including, but not limited to, a primate (*e.g.,* human), cow, sheep, goat, horse, dog, cat, rabbit, rat, or mouse. The terms "subject" and "patient" are used interchangeably herein in reference, for example, to a mammalian subject, such as a human subject.

Terms such as "treating" or "treatment" or "to treat" or "alleviating" or "to alleviate" refer to therapeutic measures that cure, slow down, lessen symptoms of, and/or halt progression of a diagnosed pathologic condition or disorder. Thus, those in need of treatment include those already diagnosed with or suspected of having the disorder. In certain aspects, a subject is successfully "treated" for cancer, *e.g.,* multiple myeloma, according to the methods of the present invention if the patient shows one or more of the following: a reduction in the number of or complete absence of cancer cells; relief of one or more symptoms associated with the specific cancer; reduced morbidity and mortality; improvement in quality of life; increased progression-free survival (PFS), disease-free survival (DFS), overall survival (OS), metastasis-free survival (MFS), complete response (CR), near complete response (nCR), stringent complete response (sCR), minor response (MR), minimal residual disease (MRD), partial response (PR), very good partial response (VGPR), stable disease (SD), a decrease in progressive disease (PD), an increased time to progression (TTP), or any combination thereof. In some aspects, the International Myeloma Working Group (IMWG) Uniform Response Criteria for Multiple Myeloma criteria can be used to determine whether the combination of an effective amount of Form A of nirogacestat dihydrobromide and the BCMA-directed therapy meets any of these particular endpoints (*e.g.,* CR, nCR, sCR, MRD).

CR for a subject having multiple myeloma can be a negative immunofixation on the serum and urine and disappearance of any soft tissue plasmacytomas and < 5% plasma cells in bone marrow.

sCR for a subject having multiple myeloma can be a CR plus normal serum free light chain (FLC) ratio and absence of clonal cells in bone marrow by immunohistochemistry or immunoflorence.

VGPR for a subject having multiple myeloma can be a serum and urine M-protein detectable by immunofixation but not on electrophoresis or > 90% reduction in serum M-protein plus urine M-protein level < 100 mg/24 h.

PD for a subject having multiple myeloma can be an increase of > 25% from lowest response value in any one or more of the following:
- serum M-component and/or (the absolute increase must be > 0.5 g/dL);
- urine M-component and/or (the absolute increase must be > 200 mg/24 h);
- only in patients without measurable serum and urine M-protein levels; the difference between involved and uninvolved FLC levels. The absolute increase must be > 10 mg/dL;
- bone marrow plasma cell percentage (the absolute percentage must be > 10%);
- definite development of new bone lesions or soft tissue plasmacytomas or definite increase in the size of existing bone lesions or soft tissue plasmacytomas;
- development of hypercalcaemia (corrected serum calcium > 11.5 mg/dL or 2.65 mmol/L) that can be attributed solely to the plasma cell proliferative disorder;

PR for a subject having multiple myeloma can be a > 50% reduction of serum M-protein and reduction in 24 hours urinary M-protein by > 90% or to < 200 mg/24 h. If the serum and urine M-protein are unmeasurable, a > 50% decrease in the difference between involved and uninvolved FLC levels can be required in place of the M-protein criteria. If the serum and urine M-protein are not measurable, and the serum free light assay can also not be measured, > 50% reduction in plasma cells can be required in place of the M-protein, provided a baseline bone marrow plasma cell percentage was > 30%. In addition, if present at baseline, a > 50% reduction in the size of soft tissue plasmacytomas can also be required.

SD for a subject having multiple myeloma can be not meeting criteria for CR, VGPR, PR, or PD.

A subject having multiple myeloma that tests MRD negative has less than one myeloma cell per million bone marrow cells.

The terms "administering," "administer," or "administration" refer to delivering one or more compounds or compositions to a subject parenterally, enterally, or topically. Illustrative examples of parenteral administration include, but are not limited to, intravenous, intramuscular, intraarterial, intrathecal, intracapsular, intraorbital, intracardiac, intradermal, intraperitoneal, transtracheal, subcutaneous, subcuticular, intraarticulare, subcapsular, subarachnoid, intraspinal and intrasternal injection and infusion. Illustrative examples of enteral administration include, but are not limited to oral, inhalation, intranasal, sublingual, and rectal administration. Illustrative examples of topical administration include, but are not limited to, transdermal and vaginal administration.

The term "effective amount" refers to an amount of a compound, formulation, material, or composition, as described herein effective to achieve a particular biological result.

The term "therapeutically effective amount" includes the amount of a compound that, when administered, is sufficient to prevent development of, or alleviate to some extent, one or more of the symptoms of a disorder, disease, or condition being treated. The term " therapeutically effective amount" also refers to the amount of a compound that is sufficient to elicit the biological or medical response of a cell, tissue, system, animal, or human, which is being sought by a researcher, veterinarian, medical doctor, or clinician.

The terms "pharmaceutically acceptable carrier," "pharmaceutically acceptable excipient," "physiologically acceptable carrier," or "physiologically acceptable excipient" refer to a pharmaceutically-acceptable material, composition, or vehicle, such as a liquid or solid filler, diluent, excipient, solvent, or encapsulating material. In one aspect, each component is "pharmaceutically acceptable" in the sense of being compatible with the other ingredients of a pharmaceutical formulation, and suitable for use in contact with the tissue or organ of humans and animals without excessive toxicity, irritation, allergic response, immunogenicity, or other problems or complications, commensurate with a reasonable benefit/risk ratio. See Remington: The Science and Practice of Pharmacy, 21st Edition, Lippincott Williams & Wilkins: Philadelphia, PA, 2005; Handbook of Pharmaceutical Excipients, 5th Edition, Rowe et al., Eds., The Pharmaceutical Press and the American Pharmaceutical Association: 2005; and Handbook of Pharmaceutical Additives, 3rd Edition, Ash and Ash Eds., Gower Publishing Company: 2007; Pharmaceutical Preformulation and Formulation, Gibson Ed., CRC Press LLC: Boca Raton, FL, 2004.

The term "inadequate expression of BCMA" as used throughout this disclosure, refers to submaximal expression levels of BCMA or equivalently the receptor density of BCMA on cancer cells as demonstrated by the fact that administration of a gamma secretase inhibitor, *e.g.,* nirogacestat dihydrobromide, can increase the expression levels or equivalently the receptor density of BCMA on cancer cells in the subject.

The term "first line of therapy" as used throughout this disclosure, refers to a treatment regimen generally accepted or recommended by the medical establishment or a regulatory authority, *e.g.,* the U.S. Food and Drug Administration or the European Medicines Agency, for the initial treatment of cancer or light chain amyloidosis in a subject. The subject having cancer or light chain amyloidosis can have previously received and/or be currently being treated for one or more unrelated diseases or disorders (*e.g.,* anxiety).

It is understood that wherever embodiments are described herein with the language "comprising," otherwise analogous embodiments described in terms of "consisting of" and/or "consisting essentially of" are also provided.

### III. Form A of Nirogacestat Dihydrobromide

The present disclosure relates combination therapies comprising Form A of nirogacestat dihydrobromide (a dihydrobromide salt of (S)-2-(((S)-6,8-difluoro-1,2,3,4-tetrahydronaphthalen-2-yl)amino)-N-(1-(2-methyl-1-(neopentylamino)propan-2-yl)-1H-imidazol-4-yl)pentanamide of Formula (I))

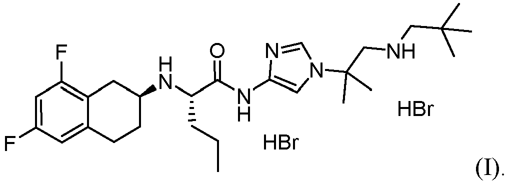

Form A of nirogacestat dihydrobromide is characterized by an XRPD pattern having peaks at 8.8 ± 0.2, 9.8 ± 0.2, and 23.3 ± 0.2 degrees two theta.

In one aspect, crystalline Form A of nirogacestat dihydrobromide is anhydrous. In another aspect, the melting point of crystalline Form A of nirogacestat dihydrobromide is about 254 °C.

In another aspect, Form A of nirogacestat dihydrobromide is characterized by an XRPD pattern having peaks at 8.8 ± 0.2, 9.8 ± 0.2, and 23.3 ± 0.2 degrees two theta when measured by Cu Kα radiation. In another aspect, Form A of nirogacestat dihydrobromide is characterized by an XRPD pattern having peaks at 8.8 ± 0.2, 9.8 ± 0.2, 23.3 ± 0.2, 25.4 ± 0.2, 28.0 ± 0.2, and 29.3 ± 0.2 degrees two theta when measured by Cu Kα radiation. In another aspect, Form A of nirogacestat dihydrobromide is characterized by an XRPD pattern having peaks at 8.8 ± 0.2, 9.8 ± 0.2, 20.0 ± 0.2, 23.3 ± 0.2, 25.4 ± 0.2, 28.0 ± 0.2, 29.3 ± 0.2, and 32.5 ± 0.2 degrees two theta when measured by Cu Kα radiation.

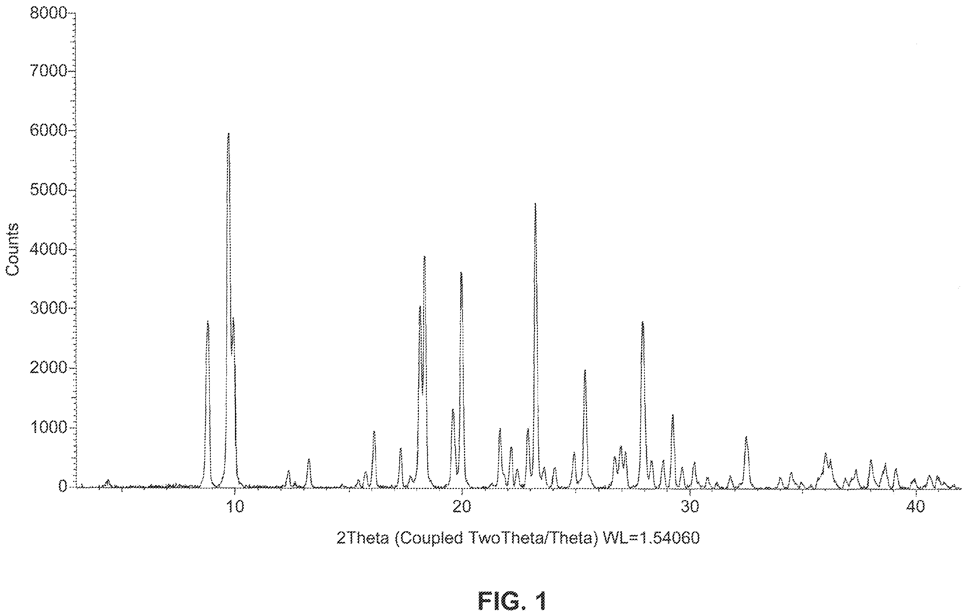

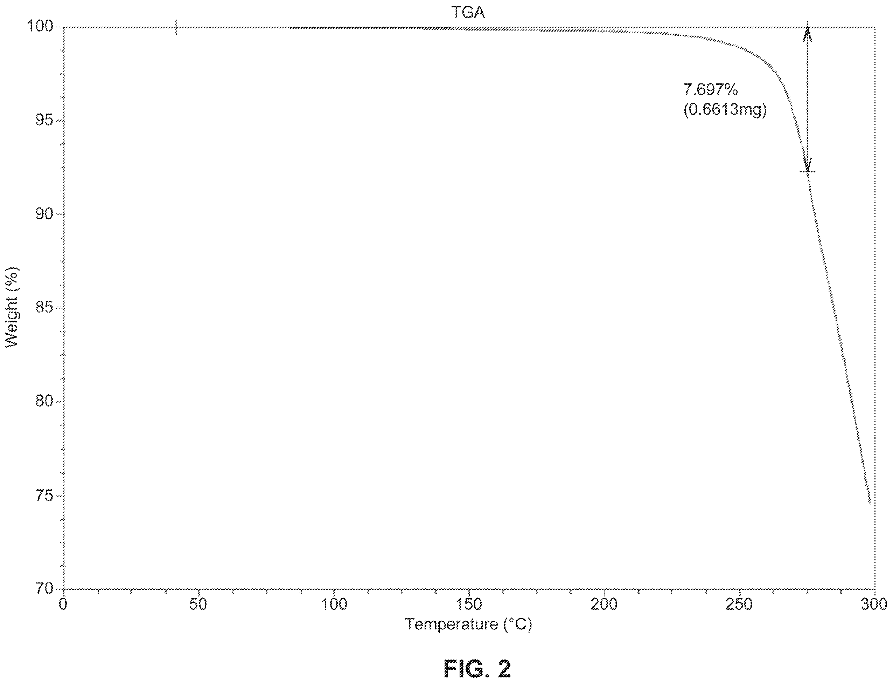

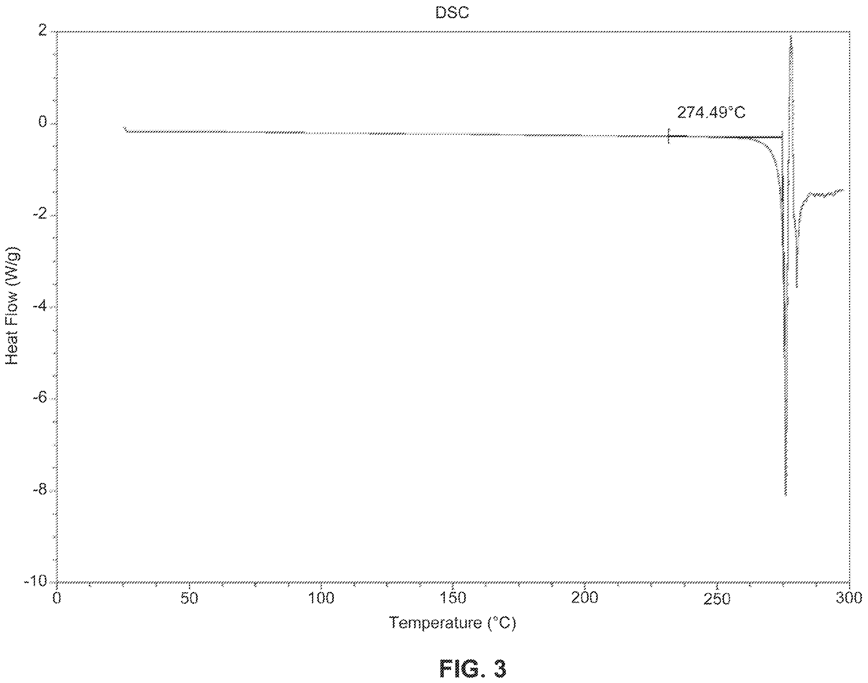

In another aspect, Form A of nirogacestat dihydrobromide is characterized by an XRPD pattern substantially as shown in Figure 1. In another aspect, Form A of nirogacestat dihydrobromide is characterized by a TGA profile substantially as shown in Figure 2. In another aspect, Form A is characterized by a DSC profile substantially as shown in Figure 3.

Form A of nirogacestat dihydrobromide can be administered to subjects via the oral, parenteral (such as subcutaneous, intravenous, intramuscular, intrasternal and infusion techniques), rectal, intranasal, topical or transdermal *(*e.g., through the use of a patch) routes. In one aspect, the Form A of nirogacestat dihydrobromide can be administered to subjects via the oral, parenteral (such as subcutaneous, intravenous, intramuscular, intrasternal and infusion techniques), rectal, intranasal, topical or transdermal (*e.g.,* through the use of a patch) routes. In one aspect, the Form A of nirogacestat dihydrobromide is orally administered. In one aspect, the Form A of nirogacestat dihydrobromide is provided in tablet form.

In one aspect, the pharmaceutical composition comprises Form A of nirogacestat dihydrobromide. In one aspect, the pharmaceutical composition is an oral tablet comprising Form A of nirogacestat dihydrobromide and a pharmaceutically acceptable carrier. In one aspect, the tablet comprises about 10 mg to about 400 mg of Form A of nirogacestat dihydrobromide. In one aspect, the tablet comprises about 10 mg, about 15 mg, about 20 mg, about 25 mg, about 30 mg, about 35 mg, about 40 mg, about 45 mg, about 50 mg, about 55 mg, about 60 mg, about 65 mg, about 70 mg, about 75 mg, about 80 mg, about 85 mg, about 90 mg, about 95 mg, about 100 mg, about 105 mg, about 110 mg, about 115 mg, about 120 mg, about 125 mg, about 130 mg, about 135 mg, about 140 mg, about 145 mg, about 150 mg, about 155 mg, about 160 mg, about 165 mg, about 170 mg, about 175 mg, about 180 mg, about 185 mg, about 190 mg, about 195 mg, about 200 mg, about 225 mg, about 250 mg, about 275 mg, about 300 mg, about 325 mg, about 350 mg, about 375 mg, or about 400 mg of Form A of nirogacestat dihydrobromide. In one aspect, the tablet comprises about 10 mg of Form A of nirogacestat dihydrobromide. In one aspect, the tablet comprises about 20 mg of Form A of nirogacestat dihydrobromide. In one aspect, the tablet comprises about 50 mg of Form A of nirogacestat dihydrobromide. In one aspect, the tablet comprises about 100 mg of Form A of nirogacestat dihydrobromide. In one aspect, the tablet comprises about 150 mg of Form A of nirogacestat dihydrobromide. In one aspect, the tablet comprises about 200 mg of Form A of nirogacestat dihydrobromide. In one aspect, the tablet comprises about 220 mg of Form A of nirogacestat dihydrobromide.

For oral administration, known carriers can be included in the pharmaceutical composition. For example, microcrystalline cellulose, sodium citrate, calcium carbonate, dicalcium phosphate and glycine can be employed along with various disintegrants such as starch (preferably corn, potato or tapioca starch), methylcellulose, alginic acid and certain complex silicates, together with granulation binders such as polyvinylpyrrolidone, sucrose, gelatin and acacia, can be included in a tablet. Additionally, lubricating agents such as magnesium stearate, sodium lauryl sulfate and talc are often useful for tabletting purposes. Solid compositions of a similar type can also be employed as fillers in gelatin capsules. Preferred materials in this connection include lactose or milk sugar as well as high molecular weight polyethylene glycols. When aqueous suspensions and/or elixirs are desired for oral administration, the active ingredient can be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration, solutions containing nirogacestat can be prepared in either sesame or peanut oil, in aqueous propylene glycol, or in sterile water or saline. The aqueous solutions should be suitably buffered (preferably pH greater than 8) if necessary and the liquid diluent first rendered isotonic with sufficient saline or glucose. These aqueous solutions are suitable for intravenous injection purposes. The oily solutions are suitable for intraarticular, intramuscular and subcutaneous injection purposes. The preparation of all these solutions under sterile conditions is readily accomplished by standard pharmaceutical techniques well known to those skilled in the art.

### IV. B-cell maturation antigen (BCMA)-directed Therapy

In some aspects, the BCMA-directed therapy, includes but is not limited to, one or more of an allogeneic chimeric antigen receptor T cell therapy, an autologous chimeric antigen receptor T cell therapy, an immunotherapy *(*e.g., a monoclonal antibody therapy), an antibody drug conjugate therapy, or a bispecific antibody therapy with dual specificity for BCMA and an immune-related target (*e.g.,* CD3). In some aspects, the BCMA-directed therapy can include at least an allogeneic chimeric antigen receptor T cell therapy. In some aspects, the BCMA-directed therapy can include at least an autologous chimeric antigen receptor T cell therapy. In some aspects, the BCMA-directed therapy can include at least an immunotherapy (*e.g.,* a monoclonal antibody therapy). In some aspects, the BCMA-directed therapy can include at least an antibody drug conjugate. In some aspects, the BCMA-directed therapy can include at least a bispecific antibody therapy with dual specificity for BCMA and an immune-related target (CD3). In some aspects the BCMA-directed therapy includes any combination of the therapies listed above.

In some aspects, the BCMA-directed therapy can be formulated for intravenous or subcutaneous administration in a liquid dosage form.

### V. Methods of Treatment

In one aspect, the combination of an effective amount of Form A of nirogacestat dihydrobromide and BCMA-directed therapy is administered to treat cancer in a subject. In some aspects, the cancer is a hematologic cancer. In one aspect, the hematologic cancer is multiple myeloma. In some aspects, the cancer is selected from a group consisting of Waldenstrom macroglobulinemia, chronic lymphocytic leukemia (CLL), diffuse large B cell lymphoma (DLBCL), follicular lymphoma (FL), Burkitt lymphoma (BL), mantle cell lymphoma (MCL), and myelogenous leukemia (ML).

In some aspects, the subject with cancer (*e.g.,* multiple myeloma) exhibits a complete response following administration of the effective amount of Form A of nirogacestat dihydrobromide and BCMA-directed therapy. In some aspects, the subject with cancer (e.g., multiple myeloma) exhibits a near complete response following administration of the effective amount of Form A of nirogacestat dihydrobromide and BCMA-directed therapy. In some aspects, the subject with cancer (*e.g*., multiple myeloma) exhibits a stringent complete response following administration of the effective amount of Form A of nirogacestat dihydrobromide and BCMA-directed therapy. In some aspects, the subject with cancer (*e.g.,* multiple myeloma) exhibits a minor response following administration of the effective amount of Form A of nirogacestat dihydrobromide and BCMA-directed therapy. In some aspects, the subject with cancer (*e.g.,* multiple myeloma) exhibits a partial response following administration of the effective amount of Form A of nirogacestat dihydrobromide and BCMA-directed therapy. In some aspects, the subject with cancer (*e.g.,* multiple myeloma) exhibits a very good partial response following administration of the effective amount of Form A of nirogacestat dihydrobromide and BCMA-directed therapy. In some aspects, the subject with cancer (*e.g.,* multiple myeloma) exhibits stable disease following administration of the effective amount of Form A of nirogacestat dihydrobromide and BCMA-directed therapy.

In one aspect, the combination of an effective amount of Form A of nirogacestat dihydrobromide and BCMA-directed therapy is administered to treat light chain amyloidosis in a subject.

In one aspect, the Form A of nirogacestat dihydrobromide is administered to the subject with cancer *(*e.g., multiple myeloma) or light chain amyloidosis before, concomitantly, or subsequently to the administering of the BCMA-directed therapy to the subject.

In one aspect, the subject with cancer (*e.g.,* multiple myeloma) or light chain amyloidosis is administered the combination therapy as the first line of therapy. In such aspect, the subject having cancer (*e.g.,* multiple myeloma) or light chain amyloidosis can have previously received and/or be currently being treated for one or more unrelated diseases or disorders (*e.g.,* anxiety).

In some aspects, combination of an effective amount of Form A of nirogacestat and the BCMA-directed therapy can be used in a combination with one or more of other known cancer treatments. In some aspects, the other known cancer treatments, include but are not limited to, a radiation therapy, a chemotherapy, a stem cell transplant an immunotherapy (*e.g*., a monoclonal antibody, such as a monoclonal antibody directed to CD38), a proteasome inhibitor, an immunomodulatory therapy, a hormone therapy, a photodynamic therapy, a targeted therapy (*e.g.,* an XPO1 inhibitor), or a combination thereof. In some aspects, the other known cancer treatments can be an immunomodulatory therapy, a proteasome inhibitor, an immunotherapy (*e.g.,* a monoclonal antibody, such as a monoclonal antibody directed to CD38), or a combination thereof. In some aspects, the other known cancer treatment can be a combination of an immunomodulatory therapy, a proteasome inhibitor, and an immunotherapy (*e.g.,* a monoclonal antibody, such as a monoclonal antibody directed to CD38).

In one aspect, the subject has cancer *(*e.g., multiple myeloma) or light chain amyloidosis after being previously treated for the cancer or light chain amyloidosis. In some aspects, the subject with cancer (*e.g.,* multiple myeloma) or light chain amyloidosis being treated with Form A of nirogacestat dihydrobromide and a BCMA-directed therapy has been previously treated for the cancer or light chain amyloidosis with one or more of a proteasome inhibitor, an immunomodulatory therapy, an immunotherapy (*e.g.,* a monoclonal antibody, such as a monoclonal antibody directed to CD38), a stem cell transplant, a chemotherapy, a targeted therapy *(e.g.,* an XPO1 inhibitor), a BCMA-directed therapy not in combination with nirogacestat to the subject, or combinations thereof. In one aspect, the subject has cancer (*e.g.,* multiple myeloma) or light chain amyloidosis after being previously treated for the cancer or light chain amyloidosis by a method comprising administration of a proteasome inhibitor to the subject. In one aspect, the subject has cancer (*e.g.,* multiple myeloma) or light chain amyloidosis after being previously treated for the cancer or light chain amyloidosis by a method comprising administration of an immunomodulatory therapy to the subject. In one aspect, the subject has cancer (*e.g.,* multiple myeloma) or light chain amyloidosis after being previously treated for the cancer or light chain amyloidosis by a method comprising administration of an immunotherapy (*e.g.,* a monoclonal antibody, such as a monoclonal antibody directed to CD38) to the subject. In one aspect, the subject has cancer (*e.g.,* multiple myeloma) or light chain amyloidosis after being previously treated for the cancer or light chain amyloidosis by a method comprising a stem cell transplant to the subject. In one aspect, the subject has cancer (*e.g.,* multiple myeloma) or light chain amyloidosis after being previously treated for the cancer or light chain amyloidosis by a method comprising administration of a chemotherapy to the subject. In one aspect, the subject has cancer (*e.g.,* multiple myeloma) or light chain amyloidosis after being previously treated for the cancer or light chain amyloidosis by a method comprising administration of a targeted therapy (*e.g.,* an XPO1 inhibitor) to the subject. In one aspect, the subject has cancer (*e.g.,* multiple myeloma) or light chain amyloidosis after being previously treated for the cancer or light chain amyloidosis by a method comprising administration of a BCMA-directed therapy not in combination with nirogacestat to the subject. In one aspect, the subject has cancer (*e.g.,* multiple myeloma) or light chain amyloidosis after being previously treated for the cancer or light chain amyloidosis by a method comprising administration of the combination of a proteasome inhibitor and an immunomodulatory therapy to the subject. In one aspect, the subject has cancer (*e.g.,* multiple myeloma) or light chain amyloidosis after being previously treated for the cancer or light chain amyloidosis by a method comprising administration of the combination of a proteasome inhibitor and an immunotherapy (*e.g.,* a monoclonal antibody, such as a monoclonal antibody directed to CD38) to the subject. In one aspect, the subject has cancer (*e.g.,* multiple myeloma) or light chain amyloidosis after being previously treated for the cancer or light chain amyloidosis by a method comprising a stem cell transplant and administration of a proteasome inhibitor to the subject. In one aspect, the subject has cancer (*e.g.,* multiple myeloma) or light chain amyloidosis after being previously treated for the cancer or light chain amyloidosis by a method comprising administration of the combination of a proteasome inhibitor and a chemotherapy to the subject. In one aspect, the subject has cancer (*e.g.,* multiple myeloma) or light chain amyloidosis after being previously treated for the cancer or light chain amyloidosis by a method comprising administration of the combination of a proteasome inhibitor and a targeted therapy (*e.g.,* an XPO1 inhibitor) to the subject. In one aspect, the subject has cancer (*e.g.,* multiple myeloma) or light chain amyloidosis after being previously treated for the cancer or light chain amyloidosis by a method comprising administration of the combination of a proteasome inhibitor and a BCMA-directed therapy not in combination with nirogacestat to the subject. In one aspect, the subject has cancer (*e.g.,* multiple myeloma) or light chain amyloidosis after being previously treated for the cancer or light chain amyloidosis by a method comprising administration of the combination of an immunomodulatory therapy and an immunotherapy (*e.g*., a monoclonal antibody, such as a monoclonal antibody directed to CD38) to the subject. In one aspect, the subject has cancer (*e.g.,* multiple myeloma) or light chain amyloidosis after being previously treated for the cancer or light chain amyloidosis by a method comprising a stem cell transplant and administration of an immunomodulatory therapy to the subject. In one aspect, the subject has cancer (*e.g.,* multiple myeloma) or light chain amyloidosis after being previously treated for the cancer or light chain amyloidosis by a method comprising administration of the combination of an immunomodulatory therapy and a chemotherapy to the subject. In one aspect, the subject has cancer (*e.g.,* multiple myeloma) or light chain amyloidosis after being previously treated for the cancer or light chain amyloidosis by a method comprising administration of the combination of an immunomodulatory therapy and a targeted therapy (*e.g.,* an XPO1 inhibitor) to the subject. In one aspect, the subject has cancer (*e.g.,* multiple myeloma) or light chain amyloidosis after being previously treated for the cancer or light chain amyloidosis by a method comprising administration of the combination of an immunomodulatory therapy and a BCMA-directed therapy not in combination with nirogacestat to the subject. In one aspect, the subject has cancer (*e.g.,* multiple myeloma) or light chain amyloidosis after being previously treated for the cancer or light chain amyloidosis by a method comprising a stem cell transplant and administration of an immunotherapy *(*e.g., a monoclonal antibody, such as a monoclonal antibody directed to CD38) to the subject. In one aspect, the subject has cancer (*e.g.,* multiple myeloma) or light chain amyloidosis after being previously treated for the cancer or light chain amyloidosis by a method comprising administration of the combination of an immunotherapy *(*e.g., a monoclonal antibody, such as a monoclonal antibody directed to CD38) and a chemotherapy to the subject. In one aspect, the subject has cancer (*e.g.,* multiple myeloma) or light chain amyloidosis after being previously treated for the cancer or light chain amyloidosis by a method comprising administration of the combination of an immunotherapy (*e.g.,* a monoclonal antibody, such as a monoclonal antibody directed to CD38) and a targeted therapy (*e.g.,* an XPO1 inhibitor) to the subject. In one aspect, the subject has cancer (*e.g.,* multiple myeloma) or light chain amyloidosis after being previously treated for the cancer or light chain amyloidosis by a method comprising administration of the combination of an immunotherapy (*e.g.,* a monoclonal antibody, such as a monoclonal antibody directed to CD38) and a BCMA-directed therapy not in combination with nirogacestat to the subject. In one aspect, the subject has cancer (*e.g.,* multiple myeloma) or light chain amyloidosis after being previously treated for the cancer or light chain amyloidosis by a method comprising a stem cell transplant and administration of a chemotherapy to the subject. In one aspect, the subject has cancer (*e.g.,* multiple myeloma) or light chain amyloidosis after being previously treated for the cancer or light chain amyloidosis by a method comprising a stem cell transplant and administration of a targeted therapy (*e.g.,* an XPO1 inhibitor) to the subject. In one aspect, the subject has cancer (*e.g.,* multiple myeloma) or light chain amyloidosis after being previously treated for the cancer or light chain amyloidosis by a method comprising a stem cell transplant and administration of a BCMA-directed therapy not in combination with nirogacestat to the subject. In one aspect, the subject has cancer (*e.g.,* multiple myeloma) or light chain amyloidosis after being previously treated for the cancer or light chain amyloidosis by a method comprising administration of the combination of a chemotherapy and a targeted therapy (*e.g.,* an XPO1 inhibitor) to the subject. In one aspect, the subject has cancer (*e.g.,* multiple myeloma) or light chain amyloidosis after being previously treated for the cancer or light chain amyloidosis by a method comprising administration of the combination of a chemotherapy and a BCMA-directed therapy not in combination with nirogacestat to the subject. In one aspect, the subject has cancer (*e.g.,* multiple myeloma) or light chain amyloidosis after being previously treated for the cancer or light chain amyloidosis by a method comprising administration of the combination of a targeted therapy (*e.g.,* an XPO1 inhibitor) and a BCMA-directed therapy not in combination with nirogacestat to the subject. In one aspect, the subject has cancer (*e.g.,* multiple myeloma) or light chain amyloidosis after being previously treated for the cancer or light chain amyloidosis by a method comprising administration of the combination of a proteasome inhibitor, an immunomodulatory therapy, and an immunotherapy (*e.g.*, a monoclonal antibody, such as a monoclonal antibody directed to CD38) to the subject. In one aspect, the subject has cancer (*e.g*., multiple myeloma) or light chain amyloidosis after being previously treated for the cancer or light chain amyloidosis by a method comprising a stem cell transplant and administration of the combination of a proteasome inhibitor and an immunomodulatory therapy to the subject. In one aspect, the subject has cancer (*e.g.,* multiple myeloma) or light chain amyloidosis after being previously treated for the cancer or light chain amyloidosis by a method comprising administration of the combination of a proteasome inhibitor, an immunomodulatory therapy, and a chemotherapy to the subject. In one aspect, the subject has cancer (*e.g.,* multiple myeloma) or light chain amyloidosis after being previously treated for the cancer or light chain amyloidosis by a method comprising administration of the combination of a proteasome inhibitor, an immunomodulatory therapy, and a targeted therapy (*e.g.,* an XPO1 inhibitor) to the subject. In one aspect, the subject has cancer (*e.g.,* multiple myeloma) or light chain amyloidosis after being previously treated for the cancer or light chain amyloidosis by a method comprising administration of the combination of a proteasome inhibitor, an immunomodulatory therapy, and a BCMA-directed therapy not in combination with nirogacestat to the subject. In one aspect, the subject has cancer (*e.g.,* multiple myeloma) or light chain amyloidosis after being previously treated for the cancer or light chain amyloidosis by a method comprising a stem cell transplant and administration of the combination of a proteasome inhibitor and an immunotherapy (*e.g.,* a monoclonal antibody, such as a monoclonal antibody directed to CD38). In one aspect, the subject has cancer (*e.g.,* multiple myeloma) or light chain amyloidosis after being previously treated for the cancer or light chain amyloidosis by a method comprising administration of the combination of a proteasome inhibitor, an immunotherapy *(*e.g., a monoclonal antibody, such as a monoclonal antibody directed to CD38), and a chemotherapy to the subject. In one aspect, the subject has cancer (*e.g.,* multiple myeloma) or light chain amyloidosis after being previously treated for the cancer or light chain amyloidosis by a method comprising administration of the combination of a proteasome inhibitor, an immunotherapy (*e.g.,* a monoclonal antibody, such as a monoclonal antibody directed to CD38), and a targeted therapy (*e.g.,* an XPO1 inhibitor) to the subject. In one aspect, the subject has cancer (*e.g.,* multiple myeloma) or light chain amyloidosis after being previously treated for the cancer or light chain amyloidosis by a method comprising administration of the combination of a proteasome inhibitor, an immunotherapy (*e.g.,* a monoclonal antibody, such as a monoclonal antibody directed to CD38), and a BCMA-directed therapy not in combination with nirogacestat to the subject. In one aspect, the subject has cancer (*e.g.,* multiple myeloma) or light chain amyloidosis after being previously treated for the cancer or light chain amyloidosis by a method comprising a stem cell transplant and administration of the combination of an immunomodulatory therapy and an immunotherapy (*e.g.,* a monoclonal antibody, such as a monoclonal antibody directed to CD38) to the subject. In one aspect, the subject has cancer (*e.g.,* multiple myeloma) or light chain amyloidosis after being previously treated for the cancer or light chain amyloidosis by a method comprising administration of the combination of an immunomodulatory therapy, an immunotherapy (*e.g.,* a monoclonal antibody, such as a monoclonal antibody directed to CD38), and a chemotherapy to the subject. In one aspect, the subject has cancer (*e.g.,* multiple myeloma) or light chain amyloidosis after being previously treated for the cancer or light chain amyloidosis by a method comprising administration of the combination of an immunomodulatory therapy, an immunotherapy (*e.g.,* a monoclonal antibody, such as a monoclonal antibody directed to CD38), and a targeted therapy *(e.g.,* an XPO1 inhibitor) to the subject. In one aspect, the subject has cancer (*e.g.,* multiple myeloma) or light chain amyloidosis after being previously treated for the cancer or light chain amyloidosis by a method comprising administration of the combination of an immunomodulatory therapy, an immunotherapy (*e.g.,* a monoclonal antibody, such as a monoclonal antibody directed to CD38), and a BCMA-directed therapy not in combination with nirogacestat to the subject. In one aspect, the subject has cancer (*e.g.,* multiple myeloma) or light chain amyloidosis after being previously treated for the cancer or light chain amyloidosis by a method comprising a stem cell transplant and administration of the combination of an immunotherapy (*e.g.,* a monoclonal antibody, such as a monoclonal antibody directed to CD38) and a BCMA-directed therapy not in combination with nirogacestat to the subject. In one aspect, the subject has cancer (*e.g.,* multiple myeloma) or light chain amyloidosis after being previously treated for the cancer or light chain amyloidosis by a method comprising administration of the combination of an immunotherapy (*e.g.,* a monoclonal antibody, such as a monoclonal antibody directed to CD38), a chemotherapy, and a BCMA-directed therapy not in combination with nirogacestat to the subject. In one aspect, the subject has cancer (*e.g.,* multiple myeloma) or light chain amyloidosis after being previously treated for the cancer or light chain amyloidosis by a method comprising administration of the combination of an immunotherapy (*e.g.,* a monoclonal antibody, such as a monoclonal antibody directed to CD38), a targeted therapy *(e.g.,* an XPO1 inhibitor), and a BCMA-directed therapy not in combination with nirogacestat to the subject. In one aspect, the subject has cancer (*e.g.,* multiple myeloma) or light chain amyloidosis after being previously treated for the cancer or light chain amyloidosis by a method comprising a stem cell transplant and administration of the combination of a chemotherapy and a targeted therapy *(e.g.,* an XPO1 inhibitor) to the subject. In one aspect, the subject has cancer (*e.g.,* multiple myeloma) or light chain amyloidosis after being previously treated for the cancer or light chain amyloidosis by a method comprising a stem cell transplant and administration of the combination of a chemotherapy and a BCMA-directed therapy not in combination with nirogacestat to the subject. In one aspect, the subject has cancer (*e.g.,* multiple myeloma) or light chain amyloidosis after being previously treated for the cancer or light chain amyloidosis by a method comprising a stem cell transplant and administration of the combination of a targeted therapy (*e.g.,* an XPO1 inhibitor) and a BCMA-directed therapy not in combination with nirogacestat to the subject. In one aspect, the subject has cancer (*e.g.,* multiple myeloma) or light chain amyloidosis after being previously treated for the cancer or light chain amyloidosis by a method comprising administration of the combination of a chemotherapy, a targeted therapy (*e.g.,* an XPO1 inhibitor), and a BCMA-directed therapy not in combination with nirogacestat to the subject. In one aspect, the subject has cancer (*e.g.,* multiple myeloma) or light chain amyloidosis after being previously treated for the cancer or light chain amyloidosis by a method comprising administration of the combination of a proteasome inhibitor, an immunomodulatory therapy, an immunotherapy (*e.g.,* a monoclonal antibody such as a monoclonal antibody directed to CD38), and a BCMA-directed therapy not in combination with nirogacestat to the subject.

In one aspect, the Form A of nirogacestat dihydrobromide prevents the cleavage of membrane bound BCMA, thereby reducing the shedding of BCMA from the surface of a BCMA positive cell in the subject. In some aspects, the Form A of nirogacestat dihydrobromide reduces about 5% to about 100% shedding of BCMA from the surface of a BCMA positive cell in the subject as compared to no administration of Form A of nirogacestat. In some aspects, the Form A of nirogacestat dihydrobromide reduces about 10% to about 100% shedding of BCMA from the surface of a BCMA positive cell in the subject as compared to no administration of Form A of nirogacestat. In some aspects, the Form A of nirogacestat dihydrobromide reduces about 15% to about 95% shedding of BCMA from the surface of a BCMA positive cell in the subject as compared to no administration of Form A of nirogacestat. In some aspects, the Form A of nirogacestat dihydrobromide reduces about 20% to about 90% shedding of BCMA from the surface of a BCMA positive cell in the subject as compared to no administration of Form A of nirogacestat. In some aspects, the Form A of nirogacestat dihydrobromide reduces about 25% to about 85% shedding of BCMA from the surface of a BCMA positive cell in the subject as compared to no administration of Form A of nirogacestat. In some aspects, the Form A of nirogacestat dihydrobromide reduces about 30% to about 80% shedding of BCMA from the surface of a BCMA positive cell in the subject as compared to no administration of Form A of nirogacestat. In some aspects, the Form A of nirogacestat dihydrobromide reduces about 35% to about 75% shedding of BCMA from the surface of a BCMA positive cell in the subject as compared to no administration of Form A of nirogacestat. In some aspects, the Form A of nirogacestat dihydrobromide reduces about 40% to about 70% shedding of BCMA from the surface of a BCMA positive cell in the subject as compared to no administration of Form A of nirogacestat. In some aspects, the Form A of nirogacestat dihydrobromide reduces about 45% to about 65% shedding of BCMA from the surface of a BCMA positive cell in the subject as compared to no administration of Form A of nirogacestat. In some aspects, the Form A of nirogacestat dihydrobromide reduces about 50% to about 60% shedding of BCMA from the surface of a BCMA positive cell in the subject as compared to no administration of Form A of nirogacestat. In some aspects, the Form A of nirogacestat dihydrobromide reduces about 50% shedding of BCMA from the surface of a BCMA positive cell in the subject as compared to no administration of Form A of nirogacestat. In some aspects, the Form A of nirogacestat dihydrobromide reduces at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or at least about 100% shedding of BCMA from the surface of a BCMA positive cell in the subject as compared to no administration of Form A of nirogacestat dihydrobromide. In some aspects, the Form A of nirogacestat dihydrobromide reduces about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% shedding of BCMA from the surface of a BCMA positive cell in the subject as compared to no administration of Form A of nirogacestat dihydrobromide.

In another aspect, the Form A of nirogacestat dihydrobromide prevents the cleavage of membrane bound BCMA, thereby reducing the levels of sBCMA in the subject. In some aspects, the Form A of nirogacestat dihydrobromide reduces about 5% to about 100% of the levels of sBCMA in the subject as compared to no administration of Form A of nirogacestat. In some aspects, the Form A of nirogacestat dihydrobromide reduces about 10% to about 100% of the levels of sBCMA in the subject as compared to no administration of Form A of nirogacestat. In some aspects, the Form A of nirogacestat dihydrobromide reduces about 15% to about 95% of the levels of sBCMA in the subject as compared to no administration of Form A of nirogacestat. In some aspects, the Form A of nirogacestat dihydrobromide reduces about 20% to about 90% of the levels of sBCMA in the subject as compared to no administration of Form A of nirogacestat. In some aspects, the Form A of nirogacestat dihydrobromide reduces about 25% to about 85% of the levels of sBCMA in the subject as compared to no administration of Form A of nirogacestat. In some aspects, the Form A of nirogacestat dihydrobromide reduces about 30% to about 80% of the levels of sBCMA in the subject as compared to no administration of Form A of nirogacestat. In some aspects, the Form A of nirogacestat dihydrobromide reduces about 35% to about 75% of the levels of sBCMA in the subject as compared to no administration of Form A of nirogacestat. In some aspects, the Form A of nirogacestat dihydrobromide reduces about 40% to about 70% of the levels of sBCMA in the subject as compared to no administration of Form A of nirogacestat. In some aspects, the Form A of nirogacestat dihydrobromide reduces about 45% to about 65% of the levels of sBCMA in the subject as compared to no administration of Form A of nirogacestat. In some aspects, the Form A of nirogacestat dihydrobromide reduces about 50% to about 60% of the levels of sBCMA in the subject as compared to no administration of Form A of nirogacestat. In some aspects, the Form A of nirogacestat dihydrobromide reduces about 50% of the levels of sBCMA in the subject as compared to no administration of Form A of nirogacestat. In some aspects, the Form A of nirogacestat dihydrobromide reduces at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or at least about 100% of the levels of sBCMA in the subject as compared to no administration of Form A of nirogacestat. In some aspects, the Form A of nirogacestat dihydrobromide reduces about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% of the levels of sBCMA in the subject as compared to no administration of Form A of nirogacestat.

In one aspect, the soluble B-cell maturation antigen (sBCMA) is measured from cell supernatants using an immunoassay (*e.g.,* ELISA), HPLC-MS or MS. In one aspect, the soluble B-cell maturation antigen (sBCMA) is measured from the serum samples of the subject using an immunoassay (*e.g*., ELISA), HPLC-MS or MS.

In one aspect, serum and supernatant samples from the subject can be analyzed by BCMA enzyme-linked immunosorbent assay (ELISA) to determine the levels of soluble BCMA in the subject. In one aspect, the serum or supernatant samples can be diluted or concentrated and the BCMA ELISA assay can be carried out according to the manufacturer's protocol. The ELISA plates can be analyzed using a plate reader.

In another aspect, the serum and supernatant samples from the subject can be analyzed by high-performance liquid chromatography coupled to a mass spectrometer (HPLC-MS) to determine the levels of soluble BCMA in the subject. In one aspect, the serum and supernatant samples from the subject can be analyzed by a mass spectrometer (MS) to determine the levels of soluble BCMA in the subject.

In another aspect, the subject administered Form A of nirogacestat dihydrobromide exhibits a greater number of BCMA-positive multiple myeloma cells after administration (post-administration) as compared with the number of BCMA-positive multiple myeloma cells prior to administration (baseline), *i.e.,* a greater percentage of BCMA-positive multiple myeloma cells post-administration as compared with baseline. In some aspects, the Form A of nirogacestat dihydrobromide increases the percentage of BCMA-positive multiple myeloma cells post-administration about 5% to about 99% as compared with baseline. In some aspects, the Form A of nirogacestat dihydrobromide increases the percentage of BCMA-positive multiple myeloma cells post-administration about 10% to about 99% as compared with baseline. In some aspects, the Form A of nirogacestat dihydrobromide increases the percentage of BCMA-positive multiple myeloma cells post-administration about 20% to about 99% as compared with baseline. In some aspects, the Form A of nirogacestat dihydrobromide increases the percentage of BCMA-positive multiple myeloma cells post-administration about 10% to about 99% as compared with baseline. In some aspects, the Form A of nirogacestat dihydrobromide increases the percentage of BCMA-positive multiple myeloma cells post-administration about 25% to about 99% as compared with baseline. In some aspects, the Form A of nirogacestat dihydrobromide increases the percentage of BCMA-positive multiple myeloma cells post-administration about 30% to about 99% as compared with baseline. In some aspects, the Form A of nirogacestat dihydrobromide increases the percentage of BCMA-positive multiple myeloma cells post-administration about 35% to about 99% as compared with baseline. In some aspects, the Form A of nirogacestat dihydrobromide increases the percentage of BCMA-positive multiple myeloma cells post-administration about 40% to about 99% as compared with baseline. In some aspects, the Form A of nirogacestat dihydrobromide increases the percentage of BCMA-positive multiple myeloma cells post-administration about 45% to about 99% as compared with baseline. In some aspects, the Form A of nirogacestat dihydrobromide increases the percentage of BCMA-positive multiple myeloma cells post-administration about 50% to about 99% as compared with baseline. In some aspects, the Form A of nirogacestat dihydrobromide increases the percentage of BCMA-positive multiple myeloma cells post-administration about 60% to about 99% as compared with baseline. In some aspects, the Form A of nirogacestat dihydrobromide increases the percentage of BCMA-positive multiple myeloma cells post-administration about 10% to about 99% as compared with baseline. In some aspects, the Form A of nirogacestat dihydrobromide increases the percentage of BCMA-positive multiple myeloma cells post-administration about 65% to about 99% as compared with baseline. In some aspects, the Form A of nirogacestat dihydrobromide increases the percentage of BCMA-positive multiple myeloma cells post-administration about 70% to about 99% as compared with baseline. In some aspects, the Form A of nirogacestat dihydrobromide increases the percentage of BCMA-positive multiple myeloma cells post-administration about 75% to about 99% as compared with baseline. In some aspects, the Form A of nirogacestat dihydrobromide increases the percentage of BCMA-positive multiple myeloma cells post-administration about 80% to about 99% as compared with baseline. In some aspects, the Form A of nirogacestat dihydrobromide increases the percentage of BCMA-positive multiple myeloma cells post-administration about 10% to about 90% as compared with baseline. In some aspects, the Form A of nirogacestat dihydrobromide increases the percentage of BCMA-positive multiple myeloma cells post-administration about 20% to about 90% as compared with baseline. In some aspects, the Form A of nirogacestat dihydrobromide increases the percentage of BCMA-positive multiple myeloma cells post-administration about 30% to about 90% as compared with baseline. In some aspects, the Form A of nirogacestat dihydrobromide increases the percentage of BCMA-positive multiple myeloma cells post-administration about 40% to about 90% as compared with baseline. In some aspects, the Form A of nirogacestat dihydrobromide increases the percentage of BCMA-positive multiple myeloma cells post-administration about 50% to about 90% as compared with baseline. In some aspects, the Form A of nirogacestat dihydrobromide increases the percentage of BCMA-positive multiple myeloma cells post-administration about 60% to about 90% as compared with baseline. In some aspects, the Form A of nirogacestat dihydrobromide increases the percentage of BCMA-positive multiple myeloma cells post-administration about 70% to about 90% as compared with baseline. In some aspects, the Form A of nirogacestat dihydrobromide increases the percentage of BCMA-positive multiple myeloma cells post-administration about 10% to about 80% as compared with baseline. In some aspects, the Form A of nirogacestat dihydrobromide increases the percentage of BCMA-positive multiple myeloma cells post-administration about 20% to about 80% as compared with baseline. In some aspects, the Form A of nirogacestat dihydrobromide increases the percentage of BCMA-positive multiple myeloma cells post-administration about 30% to about 80% as compared with baseline. In some aspects, the Form A of nirogacestat dihydrobromide increases the percentage of BCMA-positive multiple myeloma cells post-administration about 40% to about 80% as compared with baseline. In some aspects, the Form A of nirogacestat dihydrobromide increases the percentage of BCMA-positive multiple myeloma cells post-administration about 50% to about 80% as compared with baseline. In some aspects, the Form A of nirogacestat dihydrobromide increases the percentage of BCMA-positive multiple myeloma cells post-administration about 60% to about 80% as compared with baseline. In some aspects, the Form A of nirogacestat dihydrobromide increases the percentage of BCMA-positive multiple myeloma cells post-administration about 10% to about 70% as compared with baseline. In some aspects, the Form A of nirogacestat dihydrobromide increases the percentage of BCMA-positive multiple myeloma cells post-administration about 20% to about 70% as compared with baseline. In some aspects, the Form A of nirogacestat dihydrobromide increases the percentage of BCMA-positive multiple myeloma cells post-administration about 30% to about 70% as compared with baseline. In some aspects, the Form A of nirogacestat dihydrobromide increases the percentage of BCMA-positive multiple myeloma cells post-administration about 40% to about 70% as compared with baseline. In some aspects, the Form A of nirogacestat dihydrobromide increases the percentage of BCMA-positive multiple myeloma cells post-administration about 50% to about 70% as compared with baseline. In some aspects, the Form A of nirogacestat dihydrobromide increases the percentage of BCMA-positive multiple myeloma cells post-administration about 10% to about 60% as compared with baseline. In some aspects, the Form A of nirogacestat dihydrobromide increases the percentage of BCMA-positive multiple myeloma cells post-administration about 20% to about 60% as compared with baseline. In some aspects, the Form A of nirogacestat dihydrobromide increases the percentage of BCMA-positive multiple myeloma cells post-administration about 30% to about 60% as compared with baseline. In some aspects, the Form A of nirogacestat dihydrobromide increases the percentage of BCMA-positive multiple myeloma cells post-administration about 40% to about 60% as compared with baseline. In some aspects, the Form A of nirogacestat dihydrobromide increases the percentage of BCMA-positive multiple myeloma cells post-administration about 10% to about 50% as compared with baseline. In some aspects, the Form A of nirogacestat dihydrobromide increases the percentage of BCMA-positive multiple myeloma cells post-administration about 20% to about 50% as compared with baseline. In some aspects, the Form A of nirogacestat dihydrobromide increases the percentage of BCMA-positive multiple myeloma cells post-administration about 30% to about 50% as compared with baseline. In some aspects, the Form A of nirogacestat dihydrobromide increases the percentage of BCMA-positive multiple myeloma cells post-administration about 10% to about 40% as compared with baseline. In some aspects, the Form A of nirogacestat dihydrobromide increases the percentage of BCMA-positive multiple myeloma cells post-administration about 20% to about 40% as compared with baseline. In some aspects, the Form A of nirogacestat dihydrobromide increases the percentage of BCMA-positive multiple myeloma cells post-administration about 10% to about 30% as compared with baseline. In some aspects, the Form A of nirogacestat dihydrobromide increases the percentage of BCMA-positive multiple myeloma cells post-administration about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 99%.

In one aspect, the percentage of BCMA-positive multiple myeloma cells is measured by gating on negativity or positivity using flow cytometry. In one aspect, the multiple myeloma cells are extracted from the subject. In one aspect, the multiple myeloma cells are extracted from the subject through bone marrow aspiration.

In one aspect, flow cytometry assessment of bone marrow aspirate material can be performed directly on aspirate without pretreatment or following a brief ammonium chloride red blood cell lysis step. *See e.g.,* Pont, M., et al., Blood 134:1585-97 (2019). The premeasurement procedure can be adopted from established protocols next generation flow cytometric analysis for bone marrow using flow cytometer. The bone marrow aspirate can be diluted with appropriate solution and incubated under appropriate conditions. The cells can then be collected and washed with flow cytometry buffer (*e.g.,* PBS with 1% fetal bovine serum), stained with Live/Dead viability dye. Surface staining can be done with a mixture of antibodies to one or more of the group selected from CD45, CD19, CD138, CD38, CD14, CD56, CD20, CD3, CD269 (BCMA), or CD274 (PD-L1). Aliquots of normal donor PBMC cells can be stained in parallel as controls. The cells can then be washed before permeabilization/fixation using Cytofix/Cytoperm reagent for appropriate time at room temperature, washed, and stained with a mixture of antibodies to kappa and lambda immunoglobulin light chains. The samples can then be washed before resuspension in PBS and acquisition on a flow cytometer equipped with proper lasers. A minimum number of cells (*e.g.,* 5 × 10⁶ cells) can be acquired per sample. Results can be analyzed using software.

In another aspect, the Form A of nirogacestat dihydrobromide increases density of membrane bound BCMA on the surface of BCMA-positive cancer cells. In some aspects, the Form A of nirogacestat dihydrobromide increases the density of membrane bound BCMA on the surface of BCMA-positive cancer cells in the subject 2-fold, 3-fold 4-fold, 5-fold, 6-fold, 7-fold, 8-fold, 9-fold, 10-fold, 11-fold, 12-fold, 13-fold, 14-fold, 15-fold, 16-fold, 17-fold, 18-fold, 19-fold, 20-fold, 21-fold, 22-fold, 23-fold, 24-fold, 25-fold, 50-fold, 75-fold, 100-fold, 125-fold, 150-fold, 175-fold, 200-fold, 225-fold, or 250-fold.

In one aspect, surface expression of human BCMA can be determined using flow cytometry and appropriate secondary antibodies.

In another aspect, the Form A of nirogacestat dihydrobromide can be administered to enhance the activity of BCMA-directed therapy in the subject. In one aspect, the activity of the combination of the effective amount of Form A of nirogacestat dihydrobromide and the BCMA-directed therapy is measured by cancer cell killing and/or immune-mediated cancer cell killing or clearance.

In another aspect, the Form A of nirogacestat dihydrobromide enables administration of a lower dose of the BCMA-directed therapy to the subject as compared with the amount of the BCMA-directed therapy that would have been administered alone in order to achieve equal levels of efficacy (*e.g.,* one or more of the treatment endpoints discussed above (*e.g.,* CR, nCR, sCR, MRD)). In some aspects, the Form A of nirogacestat dihydrobromide enables administration of a lower dose or the same dose of BCMA-directed therapy to the subject as compared with the amount of the BCMA-directed therapy administered alone while achieving increased levels of efficacy (*e.g.,* one or more of the treatment endpoints discussed above (*e.g.,* CR, nCR, sCR, MRD)).

In one aspect, Form A of nirogacestat dihydrobromide is administered in doses ranging from about 0.1 mg to about 1000 mg daily. In one aspect, a subject is administered about 50 mg to about 500 mg of Form A of nirogacestat dihydrobromide daily. In another aspect, a subject is administered about 100 mg to about 400 mg of Form A of nirogacestat dihydrobromide daily. In another aspect, a subject is administered about 20 mg to about 220 mg of Form A of nirogacestat dihydrobromide daily. In another aspect, a subject is administered about 20 mg, about 25 mg, about 30 mg, about 40 mg, about 50 mg, about 60 mg, about 70 mg, about 75 mg, about 100 mg, about 125 mg, about 150 mg, about 175 mg, about 200 mg, about 220 mg, about 225 mg, about 250 mg, about 275 mg, about 300 mg, about 325 mg, about 350 mg, about 375 mg, or about 400 mg daily of Form A of nirogacestat dihydrobromide. The total daily dose can be provided as single or divided doses (*i.e.,* 1, 2, 3, or 4 doses per day). In one aspect, the total daily dose is provided as two doses. For example, a 300 mg or 200 mg total daily dose can be administered to a subject as two separate 150 mg or 100 mg doses, respectively. In one aspect, three tablets comprising 50 mg of Form A of nirogacestat dihydrobromide twice daily or 200 mg daily dose can be administered to a subject as two tablets comprising 50 mg of Form A of nirogacestat dihydrobromide twice daily.

In one aspect, the Form A of nirogacestat dihydrobromide is administered orally and the BCMA-directed therapy is administered intravenously or subcutaneously to the subject.

In one aspect, the subject is human.

### EXAMPLES

### A. Abbreviations and Acronyms

| | |
|---|---|
| NMR | Nuclear Magnetic Resonance Spectroscopy |
| XRPD | X-ray Powder Diffraction |
| PLM | Polarized Light Microscopy |
| TGA | Thermogravimetric Analysis |
| DSC | Differential Scanning Calorimetry |
| TG-IR | Thermogravimetric Infrared analysis |
| FE | Fast Evaporation |
| SE | Slow Evaporation |
| S/AS | Solvent/Anti-solvent |
| CP | Crash Precipitation |
| LLD | Liquid Liquid Diffusion |
| LVD | Liquid Vapor Diffusion |
| SC | Slow Cooling |
| FC | Fast Cooling |
| CC | Crash Cooling |
| LIMS | Laboratory Information Management System |
| B/E | Birefringence/Extinction |
| RT | Room/ambient Temperature |
| RH | Relative Humidity |
| VO | Vacuum Oven |
| ACN | Acetonitrile |
| CHCl₃ | Chloroform |
| DCM | Dichloromethane |
| DCE | Dichloroethane |
| DEE | Diethyl ether |
| DMA | N,N-dimethylacetamide |
| DMF | N,N-dimethylformamide |
| DMSO | Dimethyl sulfoxide |
| EtOAc | Ethyl acetate |
| EtOH | Ethanol |
| H2O | Water |
| HFIPA | Hexafluoroisopropanol |
| IPA | Isopropanol |
| IpOAc | Isopropyl acetate |
| MCH | Methyl cyclohexane |
| MeOH | Methanol |
| MEK | Methyl ethyl ketone |
| MIBK | Methyl-*iso*-butyl ketone |
| MTBE | Methyl-*tert*-butyl ether |
| NMP | N-methyl-2-pyrrolidone |
| PG | Propylene glycol |
| TFE | Trifluroethanol |
| THF | Tetrahydrofuran |

### B. Experimental Methods

### Example 1: Approximate Kinetic Solubility

Weighed samples of material were treated with aliquots of specified solvents at ambient temperature. Samples were typically sonicated between additions to facilitate dissolution. Complete dissolution was observed through visual inspection. Solubility was calculated based on the total amount of solvent added to achieve complete dissolution and may be greater than the value reported due to incremental solvent addition and the inherent kinetics of dissolution. If dissolution was not observed, values are reported as "less than". If dissolution was observed upon the first addition of solvent, values are reported as "greater than". Table 1 shows kinetic solubility of dihydrobromide salt of (s)-2-(((s)-6,8-difluoro-1,2,3,4-tetrahydronaphthalen-2-yl)amino)-n-(1-(2-methyl-1-(neopentylamino)propan-2-yl)-1h-imidazol-4-yl)pentanamide.

**Table 1**

| **Solvent** | **Solubility Estimate (a) (mg/mL)** |
|---|---|
| Acetone | <1 |
| Acetone/CHCl₃ 50/50 | <1 |
| ACN | <1 |
| ACN/CHCl₃ 50/50 | <1 |
| ACN/EtOAc 50/50 | <1 |
| Chloroform | <1 |
| DCE | <1 |
| Dioxane | '<1 |
| DMA | 8 |
| DMF | 18 |
| DMF/ACN 30/70 | <1 |
| DMF/ACN 60/40 | 3 |
| DMF/EtOAc 50/50 | <1 |
| DMF/IPA 60/40 | 5 |
| DMF/MIBK 80/20 | 4 |
| DMSO | 63 |
| DMSO/MTBE 10/90 | <1 |
| Ethylene Glycol | 7 |
| EtOAc | <1 |
| EtOH | <1 |
| EtOH/DCM 50/50 | <1 |
| Heptane/CHCl₃ 30/70 | <1 |
| MEK/DMF (b) 40/60 | 2 |
| MeOH | 19 |
| MeOH/Acetone 50/50 | 6 |
| MeOH/CHCl₃ 50/50 | 13 |
| MeOH/EtOAc 50/50 | 2 |
| MeOH/MTBE 80/20 | 7 |
| NMP | 10 |
| NMP/Acetone 85/15 | 7 |
| NMP/EtOAc 57/43 | 1 |
| PG (b) | 2 |
| TFE (anhydrous) | 10 |
| TFE/MEK 70/30 | 2 |
| THF (b) | <1 |
| THF/CHCl₃ 50/50 | 1 |
| THF/CHCl₃ 25/75 | <1 |
| THF/CHCl₃ (b) 85/15 | <1 |
| Toluene | <1 |
| Water | 7 |
| Acetone/H₂O 30/70 | 14 |
| ACN/H₂O 50/50 | 36 |
| Dioxane/H₂O (b) 50/50 | 19 |
| DMF/H₂O 50/50 | 24 |
| DMF/H₂O 30/70 | 17 |
| EtOH/H₂O 40/60 | 10 |
| IPA/H₂O 50/50 | 15 |
| MeOH/H₂O 20/80 | 9 |
| THF/H₂O 80/20 | 24 |
| THF/H₂O 90/10 | 4 |

| | |
|---|---|
| (a) Solubility estimated using solvent addition method via visual assessment of samples. Values are rounded to nearest whole number and reported as "<" if dissolution was not observed. (b) non-cGMP samples. | |

### Example 2: Stable Form and Hydrate Screen

### Method a: Trituration Experiments

Samples of dihydrobromide salt of (s)-2-(((s)-6,8-difluoro-1,2,3,4-tetrahydronaphthalen-2-yl)amino)-n-(1-(2-methyl-1-(neopentylamino)propan-2-yl)-1h-imidazol-4-yl)pentanamide were triturated at ambient or set temperature in specified solvent systems. After approximately 24 hours, solids were isolated by centrifugation using Eppendorf centrifuge tubes equipped with a 0.45 µm nylon filter. The agitation was then continued in fresh solvents for a total of ~1 and 3 weeks, after which the solids were isolated as described above, observed under polarized light and analyzed by XRPD.

### Method b: Equilibrium Solubility Testing

Equilibrium solubility of solids isolated were determined gravimetrically as follows. Measured aliquots of mother liquor solutions from the 3-week slurries were place in pre-weighed aluminum TGA pans. Subsequently, solvents were evaporated under ambient conditions or using vacuum. Remaining solids were weighed.

Table 2 shows results of stable form and hydrate screen.

**Table 2**

| **Solvent system, conditions (a)** | **Observations** | **XRPD Results** | **Solubility (mg/mL) (b)** |
|---|---|---|---|
| DMA (anhydrous) 1 week | Unknown morphology; B/E | Form A | - |
| DMA (anhydrous) 3 weeks | Unknown morphology; B/E | Form A | 27 |
| DMF/ACN (65/35) 1 week | Unknown morphology; B/E | Form A | - |
| DMF/ACN (65/35) 3 weeks | Unknown morphology; B/E | Form A | 10 |
| DMF/IPA (60/40) 1 week | Unknown morphology; B/E | Form A | - |
| DMF/IPA (60/40) 3 weeks | Unknown morphology; B/E | Form A | 13 |
| DMF/MIBK (85/15) 1 week | Unknown morphology; B/E | Form A | - |
| DMF/MIBK (85/15) 3 weeks | Unknown morphology; B/E | Form A | 19 |
| DMSO/MTBE (anhydrous) (30/70) 1 day (c) | Unknown morphology; B/E | Form A | - |
| MEK/DMF (20/80) 1 week | Unknown morphology; B/E | Form A | - |
| MEK/DMF (20/80) 3 weeks | Unknown morphology; B/E | Form A | 17 |
| MeOH (anhydrous) 2-8 °C (d) 1 week | Unknown morphology, small particles; B/E | Form A | |
| MeOH (anhydrous) 2-8 °C (d) 3 weeks | Unknown morphology + some needle-like; B/E | Form A | 13 |
| MeOH/Acetone (anhydrous) (50/50) 1 week | Unknown morphology, small particles; B/E | Form A | - |
| MeOH/Acetone (anhydrous) (50/50) 3 weeks | Unknown morphology, extremely small; B/E | Form A | 7 |
| MeOH/CHCl₃ (anhydrous) (40/60) 1 week | Unknown morphology; B/E | Form A | - |
| MeOH/CHCl₃ (anhydrous) (40/60) 3 weeks | Unknown morphology + some needle-like; B/E | Form A | 22 |
| MeOH/EtOAc (anhydrous) (70/30) 1 week | Unknown morphology, small particles; B/E | Form A | - |
| MeOH/EtOAc (anhydrous) (70/30) 3 weeks | Unknown morphology; B/E | Form A | 6 |
| MeOH/MTBE (anhydrous) (80/20) 1 week | Unknown morphology, small particles; B/E | Form A | - |
| MeOH/MTBE (anhydrous) (80/20) | Unknown morphology + some needle-like; B/E | Form A | 12 |
| 3 weeks | | | |
| NMP (anhydrous) 1 week | Unknown morphology; B/E | Form A | - |
| NMP (anhydrous) 3 weeks | Unknown morphology, very small; B/E | Form A | 50 |
| NMP/Acetone (anhydrous) (85/15) 1 week | Unknown morphology, very small particles; B/E | Form A | - |
| NMP/Acetone (anhydrous) (85/15) 3 weeks | Unknown morphology; B/E | Form A | 36 |
| NMP/EtOAc (anhydrous) (80/20) 1 week | Unknown morphology + small needles; B/E | Form A | - |
| NMP/EtOAc (anhydrous) (80/20) 3 weeks | Unknown morphology; B/E | Form A | 25 |
| PG 1 week | Unknown morphology; B/E | Form A | - |
| PG 3 weeks | Unknown morphology, extremely small; B/E | Form A | 8 |
| TFE (anhydrous) 3 weeks | Unknown morphology; B/E | Form A | - |
| TFE/MEK (anhydrous) (85/15) 1 week | Unknown morphology; B/E | Form A | - |
| TFE/MEK (anhydrous) (85/15) 3 weeks | Unknown morphology, extremely small; B/E | Form A | |
| H₂O 1 week | Unknown morphology; B/E | Form A | - |
| H₂O 3 weeks | Very small needles; B/E | Form A | 14 |
| Acetone/H₂O (50/50) 1 week | Unknown morphology, very small particles; B/E | Form A | - |
| Acetone/H₂O (50/50) 3 weeks | Unknown morphology, extremely small; B/E | Form A | 43 |
| Dioxane/H₂O (70/30) 1 week | Unknown morphology, small particles; B/E | Form A | - |
| Dioxane/H₂O (70/30) 3 weeks | Unknown morphology + some needles; B/E | Form A | 24 |
| IPA/H₂O (60/40) 1 week | Unknown morphology, very small particles; B/E | Form A | - |
| IPA/H₂O (60/40) 3 weeks | Unknown morphology, extremely small; B/E | Form A | 26 |
| EtOH/H₂O (60/40) 1 week | Unknown morphology; B/E | Form A | - |
| EtOH/H₂O (60/40) 3 weeks | Extremely small needles; B/E | Form A | 36 |
| EtOH/H₂O (60/40) 2-8 °C (d) 1 week | Unknown morphology, small particles; B/E | Form A | - |
| EtOH/H₂O (60/40) 2-8 °C (c) 3 weeks | Unknown morphology, extremely small; B/E | Form A | 33 |
| MeOH/H₂O (20/80) 1 week | Unknown morphology, small particles; B/E | Form A | - |
| MeOH/H₂O (20/80) 3 weeks | Extremely small needles; B/E | Form A | 17 |
| THF/H₂O (85/15) 1 week | Unknown morphology; B/E | Form A | - |
| THF/H₂O (85/15) 3 weeks | Unknown morphology, extremely small; B/E | Form A | 25 |

| | | | |
|---|---|---|---|
| (a) Experiments were conducted for a total of ~1 week and ~3 weeks, both with solvent replacement after ~1 day of slurrying. Solvent ratios (v/v) and duration of experiments are approximate. Experiments were performed at ambient conditions unless otherwise specified. (b) Solubility determined gravimetrically. (c) After the initial solvent exchange, there was an insufficient amount of solids to use for further slurry. (d) Conducted in a cold room. (e) non-cGMP samples. | | | |

### Example 3: Preparation of Form A of Nirogacestat Dihydrobromide

Dihydrobromide salt of (s)-2-(((s)-6,8-difluoro-1,2,3,4-tetrahydronaphthalen-2-yl)amino)-n-(1-(2-methyl-1-(neopentylamino)propan-2-yl)-1h-imidazol-4-yl)pentanamide, unless noted otherwise, was used as the starting material.

Starting materials were subjected to crystallization techniques, which are summarized below. Solids were typically isolated by vacuum filtration, observed under polarized light and analyzed by XRPD.

### Method a: Grinding Experiments

Solids were combined with small amounts of solvent and transferred to an agate milling container. An agate ball was added and the container attached to a Retsch mill. The sample was typically milled for either one cycle of twenty minutes at 30 Hz, or repacked and the cycle repeated for an additional 20 minutes.

### Method b: Slurry Experiments

Solids were suspended in specified solvents. The suspensions were then agitated at ambient or set temperature. After a given amount of time solids were isolated.

### Method c: Solvent/Anti-Solvent Precipitation

Solutions of starting material were prepared at ambient or elevated temperature and filtered using 0.2 µm nylon filters. They were then mixed with appropriate anti-solvents at elevated temperature. If no solids were observed, the samples were either cooled to ambient or sub-ambient temperatures or other crystallization techniques applied.

### Method d: Crash Precipitation

Solutions of starting material were prepared at elevated temperature in specified solvents and hot-filtered through 0.2 µm nylon filters into appropriate anti-solvents precooled on a dry ice/acetone or water/ice bath. If solids precipitated, they were immediately isolated by vacuum filtration while still cold. If the solution remained clear, the sample was either kept at sub-ambient temperatures or further crystallization techniques were applied.

### Method e: Cooling Experiments

Solutions of starting material were prepared in specified solvents at elevated temperature using a hot plate for heating. These were typically hot-filtered through a 0.2 µm nylon filter into warm receiving vials. The vials were either quickly transferred into a sub-ambient temperature bath (typically dry ice/acetone) for crash cooling (CC), removed from the hot place for fast cooling (FC) or the heat was turned off to allow for slow cooling (SC). If solids precipitated, they were isolated cold by vacuum filtration. If the solution remained clear, the sample was either kept at sub-ambient temperatures or further crystallization techniques were applied.

### Method f: Evaporation Experiments

Solutions of starting material were allowed to partially evaporate or evaporate to dryness at ambient or elevated temperature from open vials for fast evaporation (FE) or from vials covered with aluminum foil with pin holes for slow evaporation (SE). Prior to evaporation, solutions were filtered at ambient or elevated temperature using 0.2 µm nylon filters.

### Method g: Liquid-Vapor Diffusion Experiments

Solutions of starting material were prepared at ambient temperature and filtered through 0.2 µm nylon filters into receiving vials. The open vials were then placed into secondary containers with appropriate anti-solvents. The containers were sealed and left undisturbed at ambient conditions.

### Method h: Vapor Stress Experiments

Solids of starting material were transferred to vials which were placed uncapped into secondary containers with appropriate anti-solvents. The secondary containers were sealed and left undisturbed at ambient or sub-ambient conditions.

### Method i: Low Relative Humidity Stress Experiments

Solids of starting material were transferred to a vial which was placed, uncapped, into a RH jar containing P₂O₅. It was kept at ambient temperature for a specified duration.

### Method j: Drying Experiments

Solids of starting material were dried at ambient or under reduced pressure at a set temperature for a specified duration.

Table 3 summarizes the polymorph screen results for dihydrobromide salt of (s)-2-(((s)-6,8-difluoro-1,2,3,4-tetrahydronaphthalen-2-yl)amino)-n-(1-(2-methyl-1-(neopentylamino) propan-2-yl)-1h-imidazol-4-yl)pentanamide.

**Table 3**

| **Solvent system** | **Conditions (a)** | **Observations** | **XRPD Results** |
|---|---|---|---|
| ACN | Slurry, 45 °C, 2 days | Unknown morphology; B/E | Form A |
| DCE | Slurry, 45 °C, 2 days | Unknown morphology; B/E | Form A |
| DMA/DMSO (89/11) | FC attempt from 44 °C to RT (clear). Transferred to freezer, 13 days. Sonicated, FE followed by SE and then by FE at RT (small needles present in solution). Transferred back to freezer, 13 days. | Insufficient amount of solids | - |
| DMF | FC attempt from 44 °C to RT (clear). Transferred to freezer, 13 days. Sonicated, FE followed by SE and then by FE at RT (solids). Transferred back to freezer, 13 days. | Unknown morphology; B/E | Form A |
| DMF/MIBK (80/20) | SC attempt, 45 °C to RT over 2 days. Transferred to freezer, 8 days. Sonicated, FE followed by SE and then by FE at RT (small needles present in solution). Transferred back to freezer, 13 days. | Insufficient amount of solids | - |
| DMSO/MTBE (30/70) | Mother liquor; FE | Unknown morphology; B/E | Form A |
| EtOAc | Slurry, 45 °C, 2 days | Unknown morphology; B/E | Form A |
| H₂O | Slurry, RT, 4 days | Unknown morphology; B/E | Form A |
| MeOH/ACN | S/AS attempt, AS addition at 45 °C (clear); volume reduction by FE at 45 °C (gel-like solids). Added AS at 45 °C, transferred to freezer, ~1 month. | Unknown morphology; B/E | Form A, disordered |
| MeOH/CHCl₃ (anhydrous) (40/60) | Mother liquor; FE | Unknown morphology; B/E | Disordered, with peaks of Form A |
| MeOH/EtOAc (anhydrous) (70/30) | Mother liquor; FE | Glass | - |
| MeOH/MTBE | CP attempt 45 °C/sub-RT, ice bath (viscous solids). Ice bath, 2 hours, then kept at RT, 20 days. | Unknown morphology + small needles; B/E | Form A |
| TFE/MEK (anhydrous) (85/15) | Mother liquor; FE | Unknown morphology + glass; B/E | Form A |
| TFE/MEK (anhydrous) | CP attempt 45 °C/sub-RT (ice bath). Transferred to freezer, 7 days. Isolated solids while cold. | Unknown morphology; B/E | Form A |
| Dioxane/H₂O (70/30) | Mother liquor; FE | Unknown morphology + needles; B/E | Form A, possibly with X-ray amorphous content |
| IPA/H₂O (60/40) | Mother liquor; FE | Rosettes within a glassy matrix + unknown morphology; B/E | Form A, possibly with X-ray amorphous content |
| EtOH/H₂O (60/40) | Mother liquor; FE | Needles + unknown morphology; B/E | Disordered, with peaks of Form A |

| | | | |
|---|---|---|---|
| (a) Solvent ratios (v/v), temperature, and duration of experiments are approximate. Refrigerator and cold room temperature: 2-8 °C; freezer temperature: between -10 °C and -25 °C. (b) The mother liquor was obtained from the slurry at 2-8 °C. The evaporation was at ambient temperature. (c) non-cGMP samples. | | | |

Table 4 summarizes the polymorph screen results for dihydrobromide salt of (s)-2-(((s)-6,8-difluoro-1,2,3,4-tetrahydronaphthalen-2-yl)amino)-n-(1-(2-methyl-1-(neopentylamino) propan-2-yl)-1h-imidazol-4-yl)pentanamide starting from X-ray amorphous material.

**Table 4**

| **Solvent system** | **Conditions (b)** | **Observations** | **XRPD Results** |
|---|---|---|---|
| EtOH | Slurry, RT, 3 days | Unknown morphology, very small; B/E | Form A |
| DCM | Vapor stress, freezer, 6 days | Unknown morphology; B/E Solvent present. | Form H, disordered, possibly contains Form A |
| MeOH/Acetone (anhydrous) | LVD, RT | Unknown morphology + needles; B/E | Form A |
| IpOAc | Grinding; 1 cycle, 30 Hz, 20 minutes | Unknown morphology + small needles; B/E | Form A, disordered |
| Acetone/MeOH/ MCH 14/17/69 | S/AS, added AS at 30 °C. Kept at 30 °C, 3-4 hours, then cooled to RT. | Very thin needles; B/E | Form A |

| | | | |
|---|---|---|---|
| (a) All samples generated from LIMS 386797 are non-cGMP. (b) Solvent ratios (v/v), temperature, and duration of experiments are approximate. Refrigerator and cold room temperature: 2-8 °C; freezer temperature: between -10 °C and -25 °C. | | | |

### Example 4: Assessment of BCMA Expression in Human Multiple Myeloma Cell Lines Following Treatment with Form A of Nirogacestat Dihydrobromide

The BCMA-expressing multiple myeloma cells lines MM.1S, Molp-8, H929, and OPM2, and the BCMA-negative acute lymphocytic leukemia cell line REH, will be expanded in RPMI medium containing L-glutamine and 5 to 10% FBS in a humidified CO₂ incubator set to 37°C. Cells will be transferred to 96-well plates (1x10⁶ cells/mL) and cultured in the presence of increasing concentrations (0.01 nM to 3000 nM) of Form A of nirogacestat dihydrobromide or vehicle (control) in a humidified CO₂ incubator set to 37°C for 5 to 24 hours. Cells will be harvested by centrifugation for 5 minutes at 400 x g and washed with an appropriate buffer. Cells will then be suspended in 100 µL an appropriate buffer containing an anti-human BCMA antibody and stained for 30 to 60 minutes at 4°C. Cells will be washed twice with an appropriate buffer for flow cytometry analysis. The cell viability will be determined by a commercial assay as described by the manufacturer. Expression levels of BCMA (mean fluorescence intensity) will be determined by flow cytometry.

### Example 5: Assessment of sBCMA Shedding in Multiple Myeloma Cell Lines Following Treatment with Form A of Nirogacestat Dihydrobromide

The BCMA-expressing multiple myeloma cells lines MM.1S, Molp-8, H929, and OPM2, and the BCMA-negative acute lymphocytic leukemia cell line REH, will be expanded in RPMI medium containing L-glutamine and 5 to 10% FBS in a humidified CO₂ incubator set to 37°C. Cells will be transferred to 96-well plates (1x10⁶ cells/mL) and cultured in the presence of increasing concentrations (0.01 nM to 3000 nM) of Form A of nirogacestat dihydrobromide or vehicle (control) in a humidified CO₂ incubator set to 37°C for 5 to 24 hours. Cell culture media will be collected throughout and/or following a specified time and analyzed for concentration of sBCMA using a commercially available sBCMA ELISA kit according to the instructions provided by the manufacturer.

### Example 6

The BCMA-expressing multiple myeloma cells lines MM.1S, Molp-8, H929, and OPM2, and the BCMA-negative acute lymphocytic leukemia cell line REH, will be expanded in RPMI medium containing L-glutamine and 5 to 10% FBS in a humidified CO₂ incubator set to 37°C. Cells will be transferred to 96-well plates (1x10⁶ cells/mL) and cultured in the presence of a fixed dose (*e.g.,* 1 µM) of Form A of nirogacestat dihydrobromide or vehicle (control) in a humidified CO₂ incubator set to 37°C. Targeted BCMA therapies may be added a range of concentrations to evaluate the effects of the combination on the proliferation of the multiple myeloma cells in a 3-day cellular proliferation assay (*e.g.* Cell-Titre Glo).

### Example 7

Antibody-dependent cellular cytotoxicity (ADCC) activity of BCMA targeted antibodies will be determined using a BCMA directed IgG1 monoclonal antibody in combination with Form A of nirogacestat dihydrobromide. ADCC activity against BCMA-expressing multiple myeloma cells lines (*e.g.,* MM. 1S, Molp-8, RPMI8226, ARH77, GA10, LP1, L363) will be measured using commercially available assays (*e.g.,* Promega Jurkat ADCC assay) where a range of concentrations of Form A of nirogacestat dihydrobromide are combined with a range of concentrations of the BCMA targeted monoclonal antibody.

### Example 8

Bispecific cytotoxicity assays will be performed by mixing purified human CD3+ T cells and luciferase-labeled myeloma cell lines, E:T of 5:1, and serial dilutions of bispecific antibody. After 2 days of incubation, viability of cells will be assessed by OneGlo luciferase reagent (Promega).

### Example 9

T-cell dependent cellular cytotoxicity (TDCC) activity of BCMA x CD3 bispecific antibody will be determined in combination with Form A of nirogacestat dihydrobromide. Assays will be performed by mixing CD3+ T cells and luciferase-labeled multiple myeloma cell lines (*e.g.,* MM.1S, Molp-8, RPMI8226, ARH77, GA10, LP1, L363) using an effector-to-target ratio of 5 to 1. Serial dilutions of the bispecific antibody and Form A of nirogacestat dihydrobromide will result in a range of concentrations of each molecule being evaluated. After 2 days of incubation, viability of cells will be assessed using a luciferase-based assay (Promega OneGlo).

### Example 10

T-cell dependent cellular cytotoxicity (TDCC) activity of BCMA targeted chimeric antigen T-cell (CAR-T) cells will be determined in combination with Form A of nirogacestat dihydrobromide. TDCC activity against BCMA-expressing multiple myeloma cells lines (*e.g.,* MM.1S, Molp-8, RPMI8226, ARH77, GA10, LP1, L363) will be measured using custom developed TDCC assays (similar to the format described by Nazarian, A.A., et al., J. Biomol. Screen, 20:519-27 (2015)) where a range of concentrations of Form A of nirogacestat dihydrobromide will be combined with a range of BCMA targeted CAR-T cell numbers.

### Example 11

T-cell activation by BCMA targeted therapies (CAR-T cells, bispecific antibodies and monoclonal antibodies) in the presence of BCMA expressing multiple myeloma cell lines (*e.g.,* MM.1S, Molp-8, RPMI8226, ARH77, GA10, LP1, L363) will be determined in combination with Form A of nirogacestat dihydrobromide. Co-cultures of T-cells and multiple myeloma cell lines will be incubated with fixed concentrations of Form A of nirogacestat dihydrobromide. Serial dilutions of BCMA targeted therapies will be added and T-cell activation will be determined by cytokine release assays and/or flow cytometry.

In addition to the various embodiments described herein, the present disclosure includes the following aspects numbered E1 through E81. This list of aspects is presented as an exemplary list and is not necessarily part of the claimed invention.

E1. A method of treating cancer in a subject in need thereof comprising administering a combination therapy comprising an effective amount of Form A of nirogacestat dihydrobromide and a B-cell maturation antigen (BCMA)-directed therapy to the subject.

E2. The method of E1, wherein the cancer is characterized by inadequate expression of B-cell maturation antigen (BCMA).

E3. The method of E1, wherein the cancer is characterized by detectable soluble B-cell maturation antigen (BCMA) levels in a serum sample from the subject.

E4. The method of E1, wherein the cancer is a hematologic cancer.

E5. The method of E4, wherein the hematologic cancer is multiple myeloma.

E6. The method of E1, wherein the cancer is selected from a group consisting of Waldenstrom macroglobulinemia, chronic lymphocytic leukemia (CLL), diffuse large B cell lymphoma (DLBCL), follicular lymphoma (FL), Burkitt lymphoma (BL), mantle cell lymphoma (MCL), and myelogenous leukemia (ML).

E7. A method of treating light chain amyloidosis in a subject in need thereof comprising administering a combination therapy comprising an effective amount of Form A of nirogacestat dihydrobromide and a B-cell maturation antigen (BCMA)-directed therapy to the subject.

E8. The method of E1 or E7, wherein the Form A of nirogacestat dihydrobromide reduces the shedding of B-cell maturation antigen (BCMA) from the surface of a BCMA positive cell in the subject.

E9. The method of E1 or E7, wherein the Form A of nirogacestat dihydrobromide reduces the levels of soluble B-cell maturation antigen (BCMA) in the serum samples from the subject.

E10. The method of E1 or E7, wherein the Form A of nirogacestat dihydrobromide increases the percentage of B-cell maturation antigen (BCMA)-positive multiple myeloma cells in the subject.

E11. The method of E1 or E7, wherein the Form A of nirogacestat dihydrobromide increases the density of membrane bound B-cell maturation antigen (BCMA) on the surface of BCMA-positive cancer cells in the subject.

E12. The method of E1 or E7, wherein the Form A of nirogacestat dihydrobromide enhances the activity of B-cell maturation antigen (BCMA)-directed therapy in the subject.

E13. The method of E1 or E7, wherein the Form A of nirogacestat dihydrobromide enables administration of a lower dose of the B-cell maturation antigen (BCMA)-directed therapy to the subject as compared with the amount of the BCMA-directed therapy administered alone while maintaining equal levels of efficacy.

E14. The method of E1 or E7, wherein the Form A of nirogacestat dihydrobromide enables administration of a lower dose or the same dose of the B-cell maturation antigen (BCMA)-directed therapy to the subject as compared with the amount of the BCMA-directed therapy administered alone while achieving increased levels of efficacy.

E15. The method of any one of E1-E14, wherein the subject is administered the Form A of nirogacestat dihydrobromide at a dose from about 20 mg to about 220 mg.

E16. The method of any one of E1-E15, wherein the subject is administered the Form A of nirogacestat dihydrobromide at a dose from about 20 mg to about 220 mg once or twice daily.

E17. The method of any one of E1-E16, wherein the subject is administered the Form A of nirogacestat dihydrobromide at a dose of about 100 mg once or twice daily.

E18. The method of any one of E1-E16, wherein the subject is administered the Form A of nirogacestat dihydrobromide at a dose of about 50 mg once or twice daily.

E19. The method of E16, wherein the subject is administered the Form A of nirogacestat dihydrobromide at a dose from about 20 mg to about 220 mg once or twice daily for at least one week.

E20. The method of E19, wherein the subject is administered the Form A of nirogacestat dihydrobromide at a dose of about 100 mg once or twice daily for at least one week.

E21. The method of E19, wherein the subject is administered the Form A of nirogacestat dihydrobromide at a dose of about 50 mg once or twice daily for at least one week.

E22. The method of any one of E1-E21, wherein the subject is administered the Form A of nirogacestat dihydrobromide at a total daily dose of about 200 mg.

E23. The method of any one of E1-E21, wherein the subject is administered the Form A of nirogacestat dihydrobromide at a total daily dose of about 150 mg.

E24. The method of any one of E1-E21, wherein the subject is administered the Form A of nirogacestat dihydrobromide at a total daily dose of about 100 mg.

E25. The method of any one of E1-E21, wherein the subject is administered the Form A of nirogacestat dihydrobromide at a total daily dose of about 75 mg.

E26. The method of any one of E1-E21, wherein the subject is administered the Form A of nirogacestat dihydrobromide at a total daily dose of about 50 mg.

E27. The method of any one of E1-E26, wherein the Form A of nirogacestat dihydrobromide is administered to the subject before, concomitantly, or subsequently to the administering of the B-cell maturation antigen (BCMA)-directed therapy to the subject.

E28. The method of any one of E1-E27, wherein the subject is administered the combination therapy as the first line of therapy.

E29. The method of any one of E1-E27, wherein the effective amount of Form A of nirogacestat dihydrobromide and a B-cell maturation antigen (BCMA)-directed therapy are administered to the subject after the subject has been previously treated for the cancer or light chain amyloidosis.

E30. The method of E29, wherein the subject has been previously treated for the cancer or light chain amyloidosis by one or more of a proteasome inhibitor, an immunomodulatory therapy, an immunotherapy, a stem cell transplant, a chemotherapy, a targeted therapy, or a B-cell maturation antigen (BCMA)-directed therapy not in combination with nirogacestat dihydrobromide to the subject.

E31. The method of E30, wherein the immunotherapy is a monoclonal antibody.

E32. The method of E31, wherein the monoclonal antibody is directed to CD38.

E33. The method of any one of E1-E32, wherein the Form A of nirogacestat dihydrobromide is administered orally and the B-cell maturation antigen (BCMA)-directed therapy is administered intravenously or subcutaneously to the subject.

E34. The method of any one of E1-E33, wherein the B-cell maturation antigen (BCMA)-directed therapy includes one or more of an allogeneic chimeric antigen receptor T cell therapy, an autologous chimeric antigen receptor T cell therapy, an immunotherapy, an antibody drug conjugate therapy, or a bispecific antibody therapy with dual specificity for BCMA and an immune-related target.

E35. The method of E34, wherein the B-cell maturation antigen (BCMA)-directed therapy includes at least an allogeneic chimeric antigen receptor T cell therapy.

E36. The method of E34, wherein the B-cell maturation antigen (BCMA)-directed therapy includes at least an autologous chimeric antigen receptor T cell therapy.

E37. The method of E34, wherein the B-cell maturation antigen (BCMA)-directed therapy includes at least an immunotherapy.

E38. The method of E34 or E37, wherein the immunotherapy is a monoclonal antibody.

E39. The method of E34, wherein the B-cell maturation antigen (BCMA)-directed therapy includes at least an antibody drug conjugate therapy.

E40. The method of E34, wherein the B-cell maturation antigen (BCMA)-directed therapy includes at least a bispecific antibody therapy with dual specificity for BCMA and an immune-related target.

E41. The method of any one of E1-E40, wherein the Form A of nirogacestat dihydrobromide is administered in a tablet form.

E42. The method of any one of E1-E41, wherein the subject is human.

E43. Use of a combination therapy comprising an effective amount of Form A of nirogacestat dihydrobromide and a B-cell maturation antigen (BCMA)-directed therapy in treating cancer in a subject in need thereof.

E44. The use of E43, wherein the cancer is characterized by inadequate expression of B-cell maturation antigen (BCMA).

E45. The use of E43, wherein the cancer is characterized by detectable soluble B-cell maturation antigen (BCMA) levels in a serum sample from the subject.

E46. The use of E43, wherein the cancer is a hematologic cancer.

E47. The use of E46, wherein the hematologic cancer is multiple myeloma.

E48. The use of E43, wherein the cancer is selected from a group consisting of chronic lymphocytic leukemia (CLL), diffuse large B cell lymphoma (DLBCL), follicular lymphoma (FL), Burkitt lymphoma (BL), mantle cell lymphoma (MCL), and myelogenous leukemia (ML).

E49. Use of a combination therapy comprising an effective amount of Form A of nirogacestat dihydrobromide and a B-cell maturation antigen (BCMA)-directed therapy in treating light chain amyloidosis in a subject in need thereof.

E50. The use of E43 or E49, wherein the Form A of nirogacestat dihydrobromide reduces the shedding of B-cell maturation antigen (BCMA) from the surface of a BCMA positive cell in the subject.

E51. The use of E43 or E49, wherein the Form A of nirogacestat dihydrobromide reduces the levels of soluble B-cell maturation antigen (BCMA) in the subject.

E52. The use of E43 or E49, wherein the Form A of nirogacestat dihydrobromide increases the percentage of B-cell maturation antigen (BCMA)-positive multiple myeloma cells in the subject.

E53. The use of E43 or E49, wherein the Form A of nirogacestat dihydrobromide increases the density of membrane bound B-cell maturation antigen (BCMA) on the surface of BCMA-positive cancer cells in the subject.

E54. The use of E43 or E49, wherein the Form A of nirogacestat dihydrobromide enhances the activity of the B-cell maturation antigen (BCMA)-directed therapy in the subject.

E55. The use of E43 or E49, wherein the Form A of nirogacestat dihydrobromide enables use of a lower dose of the B-cell maturation antigen (BCMA)-directed therapy in the subject as compared with the amount of the BCMA-directed therapy administered alone while maintaining equal levels of efficacy.

E56. The use of E43 or E49, wherein the Form A of nirogacestat dihydrobromide enables use of a lower dose or the same dose of the B-cell maturation antigen (BCMA)-directed therapy in the subject as compared with the amount of the BCMA-directed therapy administered alone while achieving increased levels of efficacy.

E57. The use of any one of E43-E56, wherein the subject is administered the Form A of nirogacestat dihydrobromide at a dose from about 20 mg to about 220 mg.

E58. The use of any one of E43-E57, wherein the subject is administered the Form A of nirogacestat dihydrobromide at a dose from about 20 mg to about 220 mg once or twice daily.

E59. The use of any one of E43-E58, wherein the subject is administered the Form A of nirogacestat dihydrobromide at a dose of about 100 mg once or twice daily.

E60. The use of any one of E43-E58, wherein the subject is administered the Form A of nirogacestat dihydrobromide at a dose of about 50 mg once or twice daily.

E61. The use of E58, wherein the subject is administered the Form A of nirogacestat dihydrobromide at a dose from about 20 mg to about 220 mg once or twice daily for at least one week.

E62. The use of E61, wherein the subject is administered the Form A of nirogacestat dihydrobromide at a dose of about 100 mg once or twice daily for at least one week.

E63. The use of E61, wherein the subject is administered the Form A of nirogacestat dihydrobromide at a dose of about 50 mg once or twice daily for at least one week.

E64. The use of any one of E43-E63, wherein the subject is administered the Form A of nirogacestat dihydrobromide at a total daily dose of about 200 mg.

E65. The use of any one of E43-E63, wherein the subject is administered the Form A of nirogacestat dihydrobromide at a total daily dose of about 150 mg.

E66. The use of any one of E43-E63, wherein the subject is administered the Form A of nirogacestat dihydrobromide at a total daily dose of about 100 mg.

E67. The use of any one of E43-E63, wherein the subject is administered the Form A of nirogacestat dihydrobromide at a total daily dose of about 75 mg.

E68. The use of any one of E43-E63, wherein the subject is administered the Form A of nirogacestat dihydrobromide at a total daily dose of about 50 mg.

E69. The use of any one of E43-E68, wherein the Form A of nirogacestat dihydrobromide is administered to the subject before, concomitantly, or subsequently to the administering of the B-cell maturation antigen (BCMA)-directed therapy to the subject.

E70. The use of any one of E43-E69, wherein the subject is administered the combination therapy as the first line of therapy.

E71. The use of any one of E43-E69, wherein the effective amount of Form A of nirogacestat dihydrobromide and a B-cell maturation antigen (BCMA)-directed therapy are administered to the subject after the subject has been previously treated for the cancer or light chain amyloidosis.

E72. The use of E71, wherein the subject has been previously treated with one or more of a proteasome inhibitor, an immunomodulatory therapy, an immunotherapy, a stem cell transplant, a chemotherapy, a targeted therapy, or a B-cell maturation antigen (BCMA)-directed therapy not in combination with Form A of nirogacestat dihydrobromide.

E73. The use of E72, wherein the immunotherapy is a monoclonal antibody.

E74. The use of E73, wherein the monoclonal antibody is directed to CD38.

E75. The use of any one of E43-E74, wherein the B-cell maturation antigen (BCMA)-directed therapy includes one or more of an allogeneic chimeric antigen receptor T cell therapy, an autologous chimeric antigen receptor T cell therapy, an immunotherapy, an antibody drug conjugate therapy, or a bispecific antibody therapy with dual specificity for BCMA and an immune-related target.

E76. The use of E75, wherein the B-cell maturation antigen (BCMA)-directed therapy includes at least an allogeneic chimeric antigen receptor T cell therapy.

E77. The use of E75, wherein the B-cell maturation antigen (BCMA)-directed therapy includes at least an autologous chimeric antigen receptor T cell therapy.

E78. The use of E75, wherein the B-cell maturation antigen (BCMA)-directed therapy includes at least an immunotherapy.

E79. The use of E75 or E78, wherein the immunotherapy is a monoclonal antibody.

E80. The use of E79, wherein the B-cell maturation antigen (BCMA)-directed therapy includes at least an antibody drug conjugate therapy.

E81. The use of E79, wherein the B-cell maturation antigen (BCMA)-directed therapy includes at least a bispecific antibody therapy with dual specificity for BCMA and an immune-related target.

## Claims

1. A combination therapy comprising nirogacestat dihydrobromide and a B-cell maturation antigen (BCMA)-directed therapy for use in treating cancer in a subject in need thereof, wherein the nirogacestat dihydrobromide is in Form A and exhibits an XRPD pattern having peaks at 8.8 ± 0.2, 9.8 ± 0.2, and 23.3 ± 0.2 degrees two-theta.

2. The combination therapy comprising nirogacestat dihydrobromide and the BCMA-directed therapy for the use of claim 1, wherein the cancer is **characterized by** inadequate expression of BCMA;
wherein the cancer is **characterized by** detectable soluble BCMA levels in a serum sample from the subject;
wherein the cancer is a hematologic cancer, wherein optionally the hematologic cancer is multiple myeloma; or
wherein the cancer is selected from a group consisting of Waldenstrom macroglobulinemia, chronic lymphocytic leukemia (CLL), diffuse large B cell lymphoma (DLBCL), follicular lymphoma (FL), Burkitt lymphoma (BL), mantle cell lymphoma (MCL), and myelogenous leukemia (ML).

3. A combination therapy comprising nirogacestat dihydrobromide and a B-cell maturation antigen (BCMA)-directed therapy for use in treating light chain amyloidosis in a subject in need thereof, the wherein nirogacestat dihydrobromide is in Form A and exhibits an XRPD pattern having peaks at 8.8 ± 0.2, 9.8 ± 0.2, and 23.3 ± 0.2 degrees two-theta.

4. The combination therapy comprising nirogacestat dihydrobromide and the BCMA-directed therapy for the use of claim 1 or 3, wherein the Form A of nirogacestat dihydrobromide reduces the shedding of BCMA from the surface of a BCMA positive cell in the subject;
wherein the Form A of nirogacestat dihydrobromide reduces the levels of soluble BCMA in the serum samples from the subject;
wherein the Form A of nirogacestat dihydrobromide increases the percentage of BCMA-positive multiple myeloma cells in the subject;
wherein the Form A of nirogacestat dihydrobromide increases the density of membrane bound BCMA on the surface of BCMA-positive cancer cells in the subject;
wherein the Form A of nirogacestat dihydrobromide enhances the activity of BCMA-directed therapy in the subject;
wherein the Form A of nirogacestat dihydrobromide enables administration of a lower dose of the BCMA-directed therapy to the subject as compared with the amount of the BCMA-directed therapy administered alone while maintaining equal levels of efficacy; or
wherein the Form A of nirogacestat dihydrobromide enables administration of a lower dose or the same dose of the BCMA-directed therapy to the subject as compared with the amount of the BCMA-directed therapy administered alone while achieving increased levels of efficacy.

5. The combination therapy comprising nirogacestat dihydrobromide and the BCMA-directed therapy for the use of any one of claims 1-4, wherein the Form A of nirogacestat dihydrobromide is to be administered at a dose from about 20 mg to about 220 mg, preferably once or twice daily, more preferably once or twice daily for at least one week; optionally
wherein the Form A of nirogacestat dihydrobromide is to be administered at a dose of about 100 mg or of about 50 mg once or twice daily, preferably for at least one week.

6. The combination therapy comprising nirogacestat dihydrobromide and the BCMA-directed therapy for the use of any one of claims 1-5, wherein the Form A of nirogacestat dihydrobromide is to be administered at a total daily dose of about 200 mg, of about 150 mg, of about 100 mg, of about 75 mg, or of about 50 mg.

7. The combination therapy comprising nirogacestat dihydrobromide and the BCMA-directed therapy for the use of any one of claims 1-6, wherein the Form A of nirogacestat dihydrobromide is to be administered to the subject before, concomitantly, or subsequently to the administering of the BCMA-directed therapy to the subject.

8. The combination therapy comprising nirogacestat dihydrobromide and the BCMA-directed therapy for the use of any one of claims 1-7, wherein the Form A of nirogacestat dihydrobromide and the BCMA-directed therapy are to be administered as the first line of therapy.

9. The combination therapy comprising nirogacestat dihydrobromide and the BCMA-directed therapy for the use of any one of claims 1-7, wherein the Form A of nirogacestat dihydrobromide and the BCMA-directed therapy are to be administered to the subject after the subject has been previously treated for the cancer or light chain amyloidosis; optionally wherein the subject has been previously treated for the cancer or light chain amyloidosis by one or more of a proteasome inhibitor, an immunomodulatory therapy, an immunotherapy, a stem cell transplant, a chemotherapy, a targeted therapy, or a BCMA-directed therapy not in combination with nirogacestat dihydrobromide to the subject, optionally wherein the immunotherapy is a monoclonal antibody, preferably wherein the monoclonal antibody is directed to CD38.

10. The combination therapy comprising nirogacestat dihydrobromide and the BCMA-directed therapy for the use of any one of claims 1-9, wherein the Form A of nirogacestat dihydrobromide is to be administered orally and the BCMA-directed therapy is to be administered intravenously or subcutaneously to the subject.

11. The combination therapy comprising nirogacestat dihydrobromide and the BCMA-directed therapy for the use of any one of claims 1-10, wherein the BCMA-directed therapy includes one or more of an allogeneic chimeric antigen receptor T cell therapy, an autologous chimeric antigen receptor T cell therapy, an immunotherapy, an antibody drug conjugate therapy, or a bispecific antibody therapy with dual specificity for BCMA and an immune-related target.

12. The combination therapy comprising nirogacestat dihydrobromide and the BCMA-directed therapy for the use of claim 11, wherein the BCMA-directed therapy includes at least an allogeneic chimeric antigen receptor T cell therapy;
wherein the BCMA-directed therapy includes at least an autologous chimeric antigen receptor T cell therapy;
wherein the BCMA-directed therapy includes at least an antibody drug conjugate therapy; or
wherein the BCMA-directed therapy includes at least a bispecific antibody therapy with dual specificity for BCMA and an immune-related target.

13. The combination therapy comprising nirogacestat dihydrobromide and the BCMA-directed therapy for the use of claim 11, wherein the BCMA-directed therapy includes at least an immunotherapy, optionally wherein the immunotherapy is a monoclonal antibody.

14. The combination therapy comprising nirogacestat dihydrobromide and the BCMA-directed therapy for the use of any one of claims 1-13, wherein the Form A of nirogacestat dihydrobromide is to be administered in a tablet form.

15. The combination therapy comprising nirogacestat dihydrobromide and the BCMA-directed therapy for the use of any one of claims 1-14, wherein the subject is human.

## Patentansprüche

1. Kombinationstherapie, umfassend Nirogacestatdihydrobromid und eine auf B-Zell-Reifungsantigen (BCMA) gerichtete Therapie zur Verwendung bei der Behandlung von Krebs bei einem Individuum, das dies benötigt, wobei das Nirogacestatdihydrobromid in Form A vorliegt und ein XRPD-Muster zeigt, das Peaks bei 8,8 ± 0,2; 9,8 ± 0,2 und 23,3 ± 0,2 Grad zwei-Theta aufweist.

2. Kombinationstherapie, umfassend Nirogacestatdihydrobromid und die BCMA-gerichtete Therapie zur Verwendung nach Anspruch 1, wobei der Krebs durch eine unzureichende Expression von BCMA gekennzeichnet ist;
wobei der Krebs durch nachweisbare lösliche BCMA-Konzentrationen in einer Serumprobe des Individuums charakterisiert ist;
wobei der Krebs ein hämatologischer Krebs ist, wobei der hämatologische Krebs optional Multiples Myelom ist; oder
wobei der Krebs ausgewählt ist aus der Gruppe bestehend aus Morbus Waldenström, chronischer lymphatischer Leukämie (CLL), diffus großzelligem B-Zell-Lymphom (DLBCL), follikulärem Lymphom (FL), Burkitt-Lymphom (BL), Mantelzelllymphom (MCL) und myeloischer Leukämie (ML).

3. Kombinationstherapie, umfassend Nirogacestatdihydrobromid und eine auf B-Zell-Reifungsantigen (BCMA) gerichtete Therapie zur Verwendung bei der Behandlung von Leichtketten-Amyloidose bei einem Individuum, das dies benötigt, wobei das Nirogacestatdihydrobromid in Form A vorliegt und ein XRPD-Muster zeigt, das Peaks bei 8,8 ± 0,2; 9,8 ± 0,2 und 23,3 ± 0,2 Grad zwei-Theta aufweist.

4. Kombinationstherapie, umfassend Nirogacestatdihydrobromid und die BCMA-gerichtete Therapie zur Verwendung nach Anspruch 1 oder 3, wobei die Form A von Nirogacestatdihydrobromid das Ablösen von BCMA von der Oberfläche einer BCMA-positiven Zelle bei dem Individuum verringert;
wobei die Form A von Nirogacestatdihydrobromid die Konzentrationen des löslichen BCMA in den Serumproben des Individuums verringert;
wobei die Form A von Nirogacestatdihydrobromid den Anteil von BCMA-positiven multiplen Myelomzellen bei dem Individuum erhöht;
wobei die Form A von Nirogacestatdihydrobromid die Dichte des membrangebundenen BCMA auf der Oberfläche der BCMA-positiven Krebszellen bei dem Individuum erhöht;
wobei die Form A von Nirogacestatdihydrobromid die Aktivität einer BCMA-gerichteten Therapie bei dem Individuum verstärkt;
wobei die Form A von Nirogacestatdihydrobromid die Verabreichung einer niedrigeren Dosis der BCMA-gerichteten Therapie an das Individuum ermöglicht, verglichen mit der Menge der BCMA-gerichteten Therapie, die allein verabreicht wird, während gleiche Wirksamkeitsniveaus beibehalten werden; oder
wobei die Form A von Nirogacestatdihydrobromid die Verabreichung einer niedrigeren Dosis oder der gleichen Dosis der BCMA-gerichteten Therapie an das Individuum ermöglicht, verglichen mit der Menge der BCMA-gerichteten Therapie, die allein verabreicht wird, während gesteigerte Wirksamkeitsniveaus erreicht werden.

5. Kombinationstherapie, umfassend Nirogacestatdihydrobromid und die BCMA-gerichtete Therapie zur Verwendung nach einem der Ansprüche 1 bis 4, wobei die Form A von Nirogacestatdihydrobromid in einer Dosis von etwa 20 mg bis etwa 220 mg verabreicht wird, vorzugsweise einmal oder zweimal täglich, insbesondere einmal oder zweimal täglich für mindestens eine Woche; optional
wobei die Form A von Nirogacestatdihydrobromid in einer Dosis von etwa 100 mg oder von etwa 50 mg einmal oder zweimal täglich verabreicht wird, vorzugsweise für mindestens eine Woche.

6. Kombinationstherapie, umfassend Nirogacestatdihydrobromid und die BCMA-gerichtete Therapie zur Verwendung nach einem der Ansprüche 1 bis 5, wobei die Form A von Nirogacestatdihydrobromid in einer täglichen Gesamtdosis von etwa 200 mg, von etwa 150 mg, von etwa 100 mg, von etwa 75 mg oder von etwa 50 mg verabreicht wird.

7. Kombinationstherapie, umfassend Nirogacestatdihydrobromid und die BCMA-gerichtete Therapie zur Verwendung nach einem der Ansprüche 1 bis 6, wobei die Form A von Nirogacestatdihydrobromid dem Individuum vor, gleichzeitig mit oder nach der Verabreichung der BCMA-gerichteten Therapie an das Individuum verabreicht werden soll.

8. Kombinationstherapie, umfassend Nirogacestatdihydrobromid und die BCMA-gerichtete Therapie zur Verwendung nach einem der Ansprüche 1 bis 7, wobei die Form A von Nirogacestatdihydrobromid und die BCMA-gerichtete Therapie als Erstlinientherapie verabreicht werden sollen.

9. Kombinationstherapie, umfassend Nirogacestatdihydrobromid und die BCMA-gerichtete Therapie zur Verwendung nach einem der Ansprüche 1 bis 7, wobei die Form A von Nirogacestatdihydrobromid und die BCMA-gerichtete Therapie dem Individuum verabreicht werden sollen, nachdem das Individuum zuvor für den Krebs oder die Leichtketten-Amyloidose behandelt wurde; optional wobei das Individuum zuvor für den Krebs oder die Leichtketten-Amyloidose mit einem oder mehreren eines Proteasom-Inhibitors, einer immunmodulatorischen Therapie, einer Immuntherapie, einer Stammzellentransplantation, einer Chemotherapie, einer gerichteten Therapie oder einer BCMA-gerichteten Therapie nicht in Kombination mit Nirogacestatdihydrobromid an das Individuum behandelt wurde, optional wobei die Immuntherapie ein monoklonaler Antikörper ist, vorzugsweise wobei der monoklonale Antikörper auf CD38 gerichtet ist.

10. Kombinationstherapie, umfassend Nirogacestatdihydrobromid und die BCMA-gerichtete Therapie zur Verwendung nach einem der Ansprüche 1 bis 9, wobei die Form A von Nirogacestatdihydrobromid oral verabreicht werden soll und die BCMA-gerichtete Therapie intravenös oder subkutan an das Individuum verabreicht werden soll.

11. Kombinationstherapie, umfassend Nirogacestatdihydrobromid und die BCMA-gerichtete Therapie zur Verwendung nach einem der Ansprüche 1 bis 10, wobei die BCMA-gerichtete Therapie eine oder mehrere von einer allogenen chimärer Antigenrezeptor-T-Zell-Therapie, einer autologen chimärer Antigenrezeptor-T-Zell-Therapie, einer Immuntherapie, einer Antikörperarzneimittelkonjugattherapie oder einer bispezifischen Antikörpertherapie mit zweifacher Spezifität für BCMA und ein immunvermitteltes Ziel einschließt.

12. Kombinationstherapie, umfassend Nirogacestatdihydrobromid und die BCMA-gerichtete Therapie zur Verwendung nach Anspruch 11, wobei die BCMA-gerichtete Therapie mindestens eine allogene chimärer Antigenrezeptor-T-Zell-Therapie einschließt;
wobei die BCMA-gerichtete Therapie mindestens eine autologe chimärer Antigenrezeptor-T-Zell-Therapie einschließt;
wobei die BCMA-gerichtete Therapie mindestens eine Antikörperarzneimittelkonjugattherapie einschließt; oder
wobei die BCMA-gerichtete Therapie mindestens eine bispezifische Antikörpertherapie mit zweifacher Spezifität für BCMA und ein immunvermitteltes Ziel einschließt.

13. Kombinationstherapie, umfassend Nirogacestatdihydrobromid und die BCMA-gerichtete Therapie zur Verwendung nach Anspruch 11, wobei die BCMA-gerichtete Therapie mindestens eine Immuntherapie einschließt, optional wobei die Immuntherapie ein monoklonaler Antikörper ist.

14. Kombinationstherapie, umfassend Nirogacestatdihydrobromid und die BCMA-gerichtete Therapie zur Verwendung nach einem der Ansprüche 1 bis 13, wobei die Form A von Nirogacestatdihydrobromid in Tablettenform verabreicht werden soll.

15. Kombinationstherapie, umfassend Nirogacestatdihydrobromid und die BCMA-gerichtete Therapie zur Verwendung nach einem der Ansprüche 1 bis 14, wobei das Individuum ein Mensch ist.

## Revendications

1. Traitement de combinaison comprenant du dihydrobromure de nirogacestat et un traitement dirigé contre l'antigène de maturation des cellules B (BCMA), destiné à être utilisé dans le traitement d'un cancer chez un sujet qui en a besoin, dans lequel le dihydrobromure de nirogacestat se présente sous la forme A et présente un diagramme de diffraction des rayons X (XRPD) comportant des pics à 8,8 ± 0,2, 9,8 ± 0,2 et 23,3 ± 0,2 degrés deux thêta.

2. Traitement de combinaison comprenant du dihydrobromure de nirogacestat et le traitement dirigé contre le BCMA destiné à être utilisé selon la revendication 1, dans lequel le cancer est **caractérisé par** une expression insuffisante du BCMA ;
dans lequel le cancer est **caractérisé par** des taux détectables de BCMA soluble dans un échantillon de sérum provenant du sujet ;
dans lequel le cancer est un cancer hématologique, dans lequel, éventuellement, le cancer hématologique est un myélome multiple ; ou
dans lequel le cancer est choisi parmi un groupe comprenant la macroglobulinémie de Waldenström, la leucémie lymphoïde chronique (LLC), le lymphome diffus à grandes cellules B (DLBCL), le lymphome folliculaire (FL), le lymphome de Burkitt (BL), le lymphome à cellules du manteau (MCL) et la leucémie myéloïde (ML).

3. Traitement de combinaison comprenant du dihydrobromure de nirogacestat et un traitement dirigé contre l'antigène de maturation des cellules B (BCMA), destiné à être utilisé dans le traitement de l'amylose à chaînes légères chez un sujet qui en a besoin, dans lequel le dihydrobromure de nirogacestat se présente sous la forme A et présente un diagramme de diffraction des rayons X (XRPD) comportant des pics à 8,8 ± 0,2, 9,8 ± 0,2 et 23,3 ± 0,2 degrés deux thêta.

4. Traitement de combinaison comprenant du dihydrobromure de nirogacestat et un traitement dirigé contre le BCMA, destiné à être utilisé selon la revendication 1 ou la revendication 3, dans lequel la forme A du dihydrobromure de nirogacestat réduit la libération de BCMA à partir de la surface d'une cellule positive au BCMA chez le sujet ;
dans lequel la forme A du dihydrobromure de nirogacestat réduit les taux de BCMA soluble dans les échantillons de sérum provenant du sujet ;
dans lequel la forme A du dihydrobromure de nirogacestat augmente le pourcentage de cellules de myélome multiple positif au BCMA chez le sujet ;
dans lequel la forme A du dihydrobromure de nirogacestat augmente la densité de BCMA lié à la membrane à la surface des cellules cancéreuses positives au BCMA chez le sujet ;
dans lequel la forme A du dihydrobromure de nirogacestat renforce l'activité du traitement ciblé par le BCMA chez le sujet ;
dans lequel la forme A du dihydrobromure de nirogacestat permet l'administration d'une dose plus faible du traitement dirigé contre le BCMA au sujet par rapport à la quantité du traitement dirigé contre le BCMA administrée seule, tout en conservant des niveaux d'efficacité équivalents ; ou dans lequel la forme A du dihydrobromure de nirogacestat permet l'administration d'une dose plus faible ou d'une dose identique du traitement dirigé contre le BCMA au sujet par rapport à la quantité du traitement dirigé contre le BCMA administrée seule, tout en obtenant des niveaux d'efficacité accrus.

5. Traitement de combinaison comprenant du dihydrobromure de nirogacestat et le traitement dirigé contre le BCMA pour l'utilisation selon l'une quelconque des revendications 1 à 4, dans lequel la forme A du dihydrobromure de nirogacestat doit être administrée à une dose comprise entre environ 20 mg et environ 220 mg, de préférence une ou deux fois par jour, plus préférablement une ou deux fois par jour pendant au moins une semaine ; éventuellement
dans lequel la forme A du dihydrobromure de nirogacestat doit être administrée à une dose d'environ 100 mg ou d'environ 50 mg une ou deux fois par jour, de préférence pendant au moins une semaine.

6. Traitement de combinaison comprenant du dihydrobromure de nirogacestat et le traitement dirigé contre le BCMA pour l'utilisation selon l'une quelconque des revendications 1 à 5, dans lequel la forme A du dihydrobromure de nirogacestat doit être administrée à une dose quotidienne totale d'environ 200 mg, d'environ 150 mg, d'environ 100 mg, d'environ 75 mg ou d'environ 50 mg.

7. Traitement de combinaison comprenant du dihydrobromure de nirogacestat et le traitement dirigé contre le BCMA destiné à être utilisé selon l'une quelconque des revendications 1 à 6, dans lequel la forme A du dihydrobromure de nirogacestat doit être administrée au sujet avant, simultanément ou après l'administration du traitement dirigé contre le BCMA au sujet.

8. Traitement de combinaison comprenant du dihydrobromure de nirogacestat et le traitement dirigé contre le BCMA destiné à être utilisé selon l'une quelconque des revendications 1 à 7, dans lequel la forme A du dihydrobromure de nirogacestat et le traitement dirigé contre le BCMA doivent être administrés en tant que traitement de première intention.

9. Traitement de combinaison comprenant du dihydrobromure de nirogacestat et le traitement dirigé contre le BCMA destiné à être utilisé selon l'une quelconque des revendications 1 à 7, dans lequel la forme A du dihydrobromure de nirogacestat et le traitement dirigé contre le BCMA doivent être administrés au sujet après que le sujet a été préalablement traité pour le cancer ou l'amylose à chaînes légères ; dans lequel, éventuellement, le sujet a déjà été traité pour le cancer ou l'amylose à chaînes légères par un ou plusieurs des éléments suivants : un inhibiteur du protéasome, un traitement immunomodulateur, une immunothérapie, une greffe de cellules souches, une chimiothérapie, un traitement ciblé ou un traitement ciblé sur le BCMA, sans association avec le dihydrobromure de nirogacestat, dans lequel, éventuellement, l'immunothérapie consiste en un anticorps monoclonal, de préférence dans lequel l'anticorps monoclonal est dirigé contre le CD38.

10. Traitement de combinaison comprenant du dihydrobromure de nirogacestat et le traitement dirigé contre le BCMA destiné à être utilisé selon l'une quelconque des revendications 1 à 9, dans lequel la forme A du dihydrobromure de nirogacestat doit être administrée par voie orale et le traitement dirigé contre le BCMA doit être administré par voie intraveineuse ou sous-cutanée au sujet.

11. Traitement de combinaison comprenant du dihydrobromure de nirogacestat et le traitement dirigé contre le BCMA, destiné à être utilisé selon l'une quelconque des revendications 1 à 10, dans lequel le traitement dirigé contre le BCMA comprend un ou plusieurs des éléments suivants : un traitement par cellules T à récepteurs antigéniques chimériques allogéniques, un traitement par cellules T à récepteurs antigéniques chimériques autologues, une immunothérapie, un traitement par conjugué anticorps-médicament, ou un traitement par anticorps bispécifique présentant une double spécificité pour le BCMA et une cible liée au système immunitaire.

12. Traitement de combinaison comprenant du dihydrobromure de nirogacestat et le traitement dirigé contre le BCMA destiné à être utilisé selon la revendication 11, dans lequel le traitement dirigé contre le BCMA comprend au moins un traitement par cellules T à récepteurs antigéniques chimériques allogéniques ;
dans lequel le traitement dirigé contre le BCMA comprend au moins un traitement par cellules T à récepteurs antigéniques chimériques autologues ;
dans lequel le traitement dirigé contre le BCMA comprend au moins un traitement par conjugué anticorps-médicament ; ou
dans lequel le traitement dirigé contre le BCMA comprend au moins un traitement par anticorps bispécifiques présentant une double spécificité pour le BCMA et une cible liée au système immunitaire.

13. Traitement de combinaison comprenant du dihydrobromure de nirogacestat et le traitement dirigé contre le BCMA destiné à être utilisé selon la revendication 11, dans lequel le traitement dirigé contre le BCMA comprend au moins une immunothérapie, dans lequel, éventuellement, l'immunothérapie est un anticorps monoclonal.

14. Traitement de combinaison comprenant du dihydrobromure de nirogacestat et le traitement dirigé contre le BCMA destiné à être utilisé selon l'une quelconque des revendications 1 à 13, dans lequel la forme A du dihydrobromure de nirogacestat doit être administrée sous forme de comprimé.

15. Traitement de combinaison comprenant du dihydrobromure de nirogacestat et le traitement dirigé contre le BCMA destiné à être utilisé selon l'une quelconque des revendications 1 à 14, dans lequel le sujet est un être humain.
